# EUROPÄISCHE PATENTANMELDUNG

(11) **EP 4 137 117 A1**
(43) Veröffentlichungstag der Anmeldung: **22.02.2023**
(21) Anmeldenummer: 22188117.0
(22) Anmeldetag: 01.08.2022
(51) Int. Cl.: A61K 8/27, A61K 8/44, A61K 8/45, A61K 8/46, A61K 8/49, A61K 8/9789, A61Q 5/02, A61Q 5/12

(54) **HAARBEHANDLUNGSMITTEL, UMFASSEND HAARKONDITIONIERMITTEL AUF TRIMETHYLGLYCINBASIS UND EINEN AKTIVSTOFF**

(30) Priorität: 19.08.2021 DE 102021121541
(71) Anmelder: Henkel AG & Co. KGaA, 40589 Düsseldorf (DE)
(72) Erfinder: Battermann, Marlene, 21271 Asendorf (DE); Kerl, Sylvia, 25474 Bönningstedt (DE)

(57) **Zusammenfassung**

Die Erfindung betrifft Haarbehandlungsmittel, die mindestens ein durch Umsetzung von Trimethylglycin mit Polyglycerin oder Polyglycerinester und einer Säure erhältliches Reaktionsprodukt sowie einen Aktivstoff enthalten. Ferner betrifft die Erfindung auch ein Verfahren zur Verbesserung der Haarpflege, umfassend das Aufbringen der Haarbehandlungsmittel auf die Haare.

## Beschreibung

Die Erfindung betrifft Haarbehandlungsmittel, die mindestens ein durch Umsetzung von Trimethylglycin mit Polyglycerin oder Polyglycerinester und einer Säure erhältliches Reaktionsprodukt sowie einen Aktivstoff enthalten. Die Haarbehandlungsmittel weisen ein überraschend gutes Haarpflegevermögen auf und können als Haarkonditioniermittel verwendet werden. Die Erfindung betrifft auch ein Verfahren zur Verbesserung der Haarpflege, umfassend das Aufbringen der Haarbehandlungsmittel auf die Haare.

Es ist allgemein bekannt, dass häufige kosmetische Haarbehandlungen aus modischen Gründen die natürliche, altersabhängige Haarabnutzung verstärken oder beschleunigen können. Unter Haarabnutzung wird sowohl die durch Reibungsschäden wie Kämmen, Bürsten, Toupieren oder Sonneneinwirkung verursachte Schädigung der physikalischen Eigenschaften, als auch die strukturelle oder chemische Veränderung oder Schädigung der Haarfaser verstanden, welche durch Prozesse wie Färben, Bleichen oder permanente Verformung verursacht werden kann.

Die Folgen der Haarabnutzung sind oftmals am Verlust des natürlichen Haarglanzes, einem unattraktiven, stumpfen Aussehen oder einem stumpfen Haargriff erkennbar. Im Falle größerer Schäden können auch Spaltung der Haarspitzen, Abbrechen des Haares oder Auftreten kleiner Knoten im Haar beobachtet werden.

Im Handel sind kosmetische Pflegemittel verfügbar, mit denen physikalische sowie strukturelle Haarschädigungen verringert oder repariert werden können bzw. die prophylaktisch zur Vermeidung von Haarschädigungen angewendet werden können. Marktübliche Haarpflegemittel umfassen häufig einen signifikanten Anteil an Verbindungen, die entweder aus nicht nachhaltigen Quellen stammen, nur durch energetisch aufwändige Prozesse erhältlich sind und oder nicht biologisch abbaubar sind. Diese Aspekte werden von Verbrauchern zunehmend kritisch betrachtet und stattdessen mehr Wert auf einen nachhaltigeren Umgang mit Rohstoffen und Energie gelegt.

Bei der Neuentwicklung kosmetischer Produkte besteht demnach das Bestreben, vermehrt - optimalerweise ausschließlich - kosmetische Rohstoffe zu verwenden, die unter möglichst wenig Einsatz von Energie aus nachwachsenden Rohstoffen zugänglich sind und biologisch abbaubar sind. Eine Mengenreduktion oder gar ein vollständiger Austausch unerwünschter bzw. problematischer Stoffe kann jedoch nur dann vorgenommen werden, wenn Alternativen zur Verfügung stehen, welche die für den Anwendungszweck gewünschten Eigenschaften ebenfalls aufweisen.

In den vergangenen Jahren wurden zahlreiche Versuche unternommen, übliche kationische Pflegestoffe wie kationische Polymere und/oder kationische Tenside vollständig oder zumindest teilweise durch alternative Pflegewirkstoffe aus nachwachsenden Rohstoffen zu ersetzen, welche des Weiteren biologisch abbaubar sind.

In DE 102009048299A1 wurden beispielsweise Haarbehandlungsmittel zur Reinigung und Pflege von Haaren vorgeschlagen, die neben Tensiden Proteolipide als haarpflegenden Wirkstoff enthalten. Die FR 3099058A1 betrifft ein kosmetisches Verfahren, bei dem Trimethylglycinestersalze als haarpflegende Tenside im Emulsionszusammensetzungen offenbart werden. Diese sind durch eine Veresterung von Trimethylglycin mit C₁₄₋₂₄-Fettalkoholen in Anwesenheit einer Säure erhältlich.

Für die Bereitstellung leichterer, vielseitigerer und nachhaltigerer Haarpflegeformulierungen sind jedoch wasserlösliche oder in Wasser dispergierbare Pflegestoffe wünschenswert, die in wässrigen Medien nicht stabilisiert oder durch Zusatz organischer Lösungsmittel gelöst werden müssen. Der oder die Pflegestoff(e) sollten zudem ein hohes Pflegepotential aufweisen, damit auf die Verwendung von Pflegestoffen aus nicht nachhaltigen Quellen ganz oder teilweise verzichtet werden kann. Ferner sollte(n) der oder die Pflegestoff(e) biologisch abbaubar sein. Ziel der vorliegenden Erfindung war die Bereitstellung solcher Haarpflegeformulierungen.

Die Aufgabe wird erfindungsgemäß durch ein Haarbehandlungsmittel gelöst, umfassend:
a) mindestens ein Reaktionsprodukt, welches durch Umsetzung von
   i) Polyglycerin oder einem Polyglycerinester mit
   ii) Trimethylglycin und
   iii) mindestens einer Säure erhältlich ist, sowie
b) mindestens einen Aktivstoff.

Erfindungsgemäße Haarbehandlungsmittel können in jeder beliebigen Form vorliegen, vorzugsweise liegen sie als Shampoo, als Haarkonditioniermittel, wie eine Haarspülung, als Kur, als Essence, als Lotion, als Serum, als Pulver, als Wachs, als Gel, als Feststücke (Solids), als Spray oder als Haarspitzenfluid vor.

Unter einem "Aktivstoff" wird im Rahmen der vorliegenden Erfindung ein Stoff verstanden, der unmittelbar an Haut oder Haaren eine Wirkung entfaltet. Die Wirkung wird durch die Komponente a) verstärkt.

Es wurde gefunden, dass die Kombination der Komponente a) auf Trimethylglycinbasis und mindestens eines Aktivstoffes als Komponente b) den Haarbehandlungsmitteln außergewöhnlich gute Pflegeeigenschaften verleiht. Insbesondere weisen Haare, die mit hierin definierten Haarbehandlungsmitteln behandelt wurden, verbesserte antistatische Eigenschaften, erhöhte Weichheit und Geschmeidigkeit sowie ein bessere Kämmbarkeit und/oder Entwirrbarkeit auf. Ferner zeigt die Kombination der Komponenten einen positiven Effekt auf die Grenzflächenspannung des Haares, der durch Kontaktwinkelmessung nachweisbar ist.

Weiterhin vorteilhaft ist, dass die Pflegestoffe a) und b) wasserlöslich sind und sich problemlos in wässrige Haarbehandlungsmittel einarbeiten lassen. Dadurch können leichte, bei Bedarf auch transparente, Haarbehandlungsmittel zur Verfügung gestellt werden, denen zum Erzielen eines optimalen Pflegepotentials weder synthetische (polymere) Stabilisierungsmittel noch weitere Pflegestoffe notwendigerweise hinzugefügt werden müssen. Für den Fall, dass die erfindungsgemäßen Haarbehandlungsmittel dennoch weitere Pflegestoffe enthalten, so kann deren Einsatzmenge gegenüber handelsüblichen Zusammensetzungen erheblich reduziert werden.

Die vorliegende Erfindung stellt auch ein Haarbehandlungsmittel bereit, enthaltend
A) mindestens eine Verbindung der allgemeinen Formel (I)

   R¹(OCH₂CH(OR²)CH₂)ₓ-O-R³ (I)

   in der
   ∘ R¹, R², R³ unabhängig voneinander für -H, -C(O)-CH₂-N⁺(CH₃)₃ oder für einen geradkettigen oder verzweigten, gesättigten oder ungesättigten, -OH oder NH₂-Substituenten tragenden C₆-C₃₀-Acylrest stehen,
   ∘ x für eine Zahl von 1,1 bis 30 steht,
   ∘ wobei mindestens einer der Reste R¹, R², R³ für die Gruppierung -C(O)CH₂-N⁺(CH₃)₃ steht, und
   ∘ wobei Formel (I), entsprechend der Anzahl der darin enthaltenen positiven Ladungen, mindestens ein physiologisch akzeptables Anion A⁻ enthält, und
B) mindestens einen Aktivstoff.

Die vorliegende Erfindung stellt auch ein Verfahren zur Verbesserung der Haarpflege bereit, umfassend das Aufbringen eines hierin definierten Haarbehandlungsmittels auf das Haar.

Die vorliegende Erfindung stellt auch die Verwendung eines hierin definierten Haarbehandlungsmittels zur Verbesserung der Haarpflege, insbesondere zur Verbesserung der antistatischen Eigenschaften, der Weichheit, der Kämmbarkeit und/oder Entwirrbarkeit von Haaren bereit.

Die oben zusammengefasste Erfindung wird nun detaillierter dargestellt.

Sofern nicht anders angegeben, sind alle in % ausgedrückten Mengen Gew.-% und beziehen sich auf das Gesamtgewicht des Haarbehandlungsmittels.

Geeignete Polyglycerine a)i) im Sinne der vorliegenden Erfindung stellen beispielsweise Eigenkondensationsprodukte von Glycerin mit 1,1 bis 30 Glycerineinheiten dar. Das zur Kondensation verwendete Glycerin stammt dabei vorzugsweise ausschließlich aus natürlichen Quellen, wie pflanzlichen Ölen.

Vorzugsweise weisen Polyglycerine a)i) einen mittleren Kondensationsgrad von 1,1 bis 10, mehr bevorzugt 1,1 bis 6, besonders bevorzugt 2 bis 4 und insbesondere 3 auf.

Besonders bevorzugte Polyglycerine a)i) im Sinne der vorliegenden Erfindung sind die unter den INCI-Bezeichnungen Polyglycerine-2, Polyglycerine-3, Polyglycerine-4, Polyglycerine-5, Polyglycerine-6, Polyglycerine-7, Polyglycerine-8, Polyglycerine-9, Polyglycerine-10 bekannte Verbindungen. Ganz besonders bevorzugt sind Polyglycerine-2, Polyglycerine-3, Polyglycerine-4, Polyglycerine-5 und/oder Polyglycerine-6 und insbesondere bevorzugt ist Polyglycerine-3. Erfindungsgemäß geeignete Polyglycerine a)i. sind im Handel erhältlich, beispielsweise von der Firma Solvay.

Geeignete Polyglycerinester a)i) im Sinne der vorliegenden Erfindung sind vorzugsweise Polyglycerinester a)i), die aus Polyglycerin und mit 1,1 bis 30 Glycerineinheiten und mindestens einer geradkettigen oder verzweigten, gesättigten oder ungesättigten C₆-C₃₀-Carbonsäure gebildet werden. Die Polyglycerineinheit ist dabei wie zuvor beschrieben definiert.

Unter bevorzugten Polyglycerinestern a)i) im Sinne der vorliegenden Erfindung werden demnach Monoester, Diester, Triester, Tertraester usw. aus 1,1 bis 30 Glycerineinheiten umfassendem Polyglycerin mit Behensäure, Caprinsäure, Caprylsäure, Olivensäuren, Kokossäuren, Linolsäure, Dilinolsäure, Myristinsäure, Stearinsäure, Isostearinsäure, Hydroxystearinsäure, Laurinsäure, Ölsäure, Palmitinsäure, Erucasäure, Palmsäuren, Ricinolsäure, Decansäure, Undecylensäure oder Mischungen davon verstanden.

Besonders bevorzugte Polyglycerinester a)i) im Sinne der vorliegenden Erfindung können ausgewählt sein aus unter den INCI-Bezeichnungen Polyglyceryl-2 Caprate, Polyglyceryl-2 Dicaprate, Polyglyceryl-3 Caprate, Polyglyceryl-3 Tricaprate, Polyglyceryl-4 Caprate, Polyglyceryl-5 Caprate, Polyglyceryl-6 Caprate, Polyglyceryl-10 Caprate, Polyglyceryl-2 Caprylate, Polyglyceryl-2 Dicaprylate, Polyglyceryl-3 Caprylate, Polyglyceryl-3 Tricaprylate, Polyglyceryl-4 Caprylate, Polyglyceryl-5 Caprylate, Polyglyceryl-6 Caprylate, Polyglyceryl-10 Caprylate, Polyglyceryl-2 Cocoate, Polyglyceryl-2 Dicocoate, Polyglyceryl-3 Cocoate, Polyglyceryl-3 Triccocoate, Polyglyceryl-4 Cocoate, Polyglyceryl-5 Cocoate, Polyglyceryl-6 Cocoate, Polyglyceryl-10 Cocoate, Polyglyceryl-2 Stearate, Polyglyceryl-2 Distearate, Polyglyceryl-3 Stearate, Polyglyceryl-3 Tristearate, Polyglyceryl-4 Stearate, Polyglyceryl-5 Stearate, Polyglyceryl-6 Stearate, Polyglyceryl-10 Stearate, Polyglyceryl-2 Isostearate, Polyglyceryl-2 Diisostearate, Polyglyceryl-3 Isostearate, Polyglyceryl-3 Triisostearate, Polyglyceryl-4 Isostearate, Polyglyceryl-5 Isostearate, Polyglyceryl-6 Isostearate, Polyglyceryl-10 Isostearate, Polyglyceryl-2 Ricinoleate, Polyglyceryl-2 Diricinoleate, Polyglyceryl-3 Ricinoleate, Polyglyceryl-3 Triricinoleate, Polyglyceryl-3 Polyricinoleate, Polyglyceryl-4 Ricinoleate, Polyglyceryl-5 Ricinoleate, Polyglyceryl-6 Ricinoleate, Polyglyceryl-10 Ricinoleate, Polyglyceryl-2 Oleate, Polyglyceryl-2 Dioleate, Polyglyceryl-3 Oleate, Polyglyceryl-3 Trioleate, Polyglyceryl-4 Oleate, Polyglyceryl-5 Oleate, Polyglyceryl-6 Oleate, Polyglyceryl-10 Oleate bekannten Verbindungen. Insbesondere bevorzugt ist Polyglyceryl-3-oleate). Erfindungsgemäß geeignete Polyglycerinester a)i. sind im Handel erhältlich, beispielsweise von der Firma Evonik.

Trimethylglycin a)ii) (INCI-Bezeichnung: Betaine) ist eine in vielen Pflanzen (Broccoli, Spinat, Zuckerrüben, Quinoa, Roggen, Süßkartoffel) vorkommende Verbindung, die sich beispielsweise als Nebenprodukt bei der Zuckerherstellung gewinnen lässt.

Trimethylglycin selbst wurde in der Vergangenheit bereits in kosmetischen Mitteln eingesetzt, unter anderem als haarkonditionierende Komponente, insbesondere feuchtigkeitsspendender oder die Haarfasern verstärkender Wirkstoff.

Als Säure a)iii) kommen anorganische oder organische Säuren, vorzugsweise organische Säuren, aus der Gruppe der geradkettigen oder verzweigten, gesättigten oder ungesättigten C₁-C₂₄-Mono-, Di- oder Tricarbonsäuren, welche eine oder mehrere Hydroxylgruppen als Substituenten tragen können, und/oder aromatische Säuren in Frage.

Besonders bevorzugte Säuren a)iii) sind ausgewählt aus Ameisensäure, Essigsäure, Propionsäure, Caprylsäure, Caprinsäure, Benzoesäure, Zitronensäure, Oxalsäure, Weinsäure, (Poly)acrylsäure, Fumarsäure, Maleinsäure, Itaconsäure, Glykolsäure, Gluconsäure, Äpfelsäure, Milchsäure, Mandelsäure, Tiglinsäure, Succinsäure oder Ascorbinsäure. Ganz besonders bevorzugt sind ausschließlich aus natürlichen Quellen stammende Säuren, wie beispielsweise aus Pflanzen erhältliche Essigsäure.

In einer besonders bevorzugten Ausführungsform enthalten erfindungsgemäße Haarbehandlungsmittel als Komponente a) mindestens ein Reaktionsprodukt aus
i) Polyglycerine-3 und/oder Polyglyceryl-3 Oleate,
ii) Trimethylglycin und
iii) Essigsäure.
wobei die Ausgangsstoffe i) bis iii) ausschließlich aus natürlichen Quellen stammen.

Ein Gegenstand der Erfindung ist auch ein Haarbehandlungsmittel, enthaltend
A) mindestens eine Verbindung der allgemeinen Formel (I)

   R¹(OCH₂CH(OR²)CH₂)ₓ-O-R³ (I)

   in der
   ∘ R¹, R², R³ unabhängig voneinander für -H, -C(O)-CH₂-N⁺(CH₃)₃ oder für einen geradkettigen oder verzweigten, gesättigten oder ungesättigten, -OH oder NH₂-Substituenten tragenden C₆-C₃₀-Acylrest stehen, und
   ∘ x für eine Zahl von 1,1 bis 30 steht,
   ∘ wobei mindestens einer der Reste R¹, R², R³ für die Gruppierung -C(O)CH₂-N⁺(CH₃)₃ steht, und
   ∘ wobei Formel (I), entsprechend der Anzahl der darin enthaltenen positiven Ladungen, mindestens ein physiologisch akzeptables Anion A⁻ enthält, und
B) mindestens einen Aktivstoff.

Bevorzugt sind Verbindungen A) gemäß Formel (I), in der
- einer der Reste R¹, R³ für -C(O)-CH₂-N⁺(CH₃)₃ und der andere für -H, -C(O)-CH₂-N⁺(CH₃)₃ oder für einen geradkettigen oder verzweigten, gesättigten oder ungesättigten, -OH oder NH₂-Substituenten tragenden C₆-C₂₀-Acylrest,
- R² für -H, und
- x für eine Zahl von 1,1 bis 10 steht.

Beispiele für bevorzugte Verbindungen A) der Formel (I) sind der nachfolgenden Tabelle zu entnehmen:

| x | R¹ | R² | R3 |
|---|---|---|---|
| 1,1-10 | -H | -H | -C(O)-CH₂-N⁺(CH₃)₃ |
| 1,1-10 | -C(O)-CH₂-N⁺(CH₃)₃ | -H | -H |
| 1,1-10 | -C(O)-CH₂-N⁺(CH₃)₃ | -H | -C(O)-CH₂-N⁺(CH₃)₃ |
| 1,1-10 | C₆-C₂₀-Acyl* | -H | -C(O)-CH₂-N⁺(CH₃)₃ |
| 1,1-10 | -C(O)-CH₂-N⁺(CH₃)₃ | -H | C₆-C₂₀-Acyl* |

| | | | |
|---|---|---|---|
| * unter "C₆-C₂₀-Acylrest" werden in der Tabelle geradkettige oder verzweigte, gesättigte oder ungesättigte, -OH oder NH₂-Substituenten tragende Acylreste mit 6 bis 20 Kohlenstoffatomen verstanden | | | |

Mehr bevorzugt sind Verbindungen A) gemäß Formel (I), in der
- einer der Reste R¹, R³ für -C(O)-CH₂-N⁺(CH₃)₃ und der andere für -H, -C(O)-CH₂-N⁺(CH₃)₃ oder für Heptanoat, Caprylat, Nonanoat, Caprat, Undecylat, Laurat, Tridecanoat, Myristat, Pentadecanoat, Palmitat, Heptadecanoat, Stearat, Isostearat, Oleat, Ricinoleat, Nonadecanoat, Arachidat, Behenat,
- R² für -H, und
- x für eine Zahl von 1,1 bis 6, vorzugsweise von 2 bis 4 und insbesondere für 3 steht.

Beispiele für mehr bevorzugte Verbindungen A) der Formel (I) sind der nachfolgenden Tabelle zu entnehmen:

| x | R¹ | R² | R³ |
|---|---|---|---|
| 1,1-6 | -H | -H | -C(O)-CH₂-N⁺(CH₃)₃ |
| 1,1-6 | -C(O)-CH₂-N⁺(CH₃)₃ | -H | -H |
| 1,1-6 | -C(O)-CH₂-N⁺(CH₃)₃ | -H | -C(O)-CH₂-N⁺(CH₃)₃ |
| 1,1-6 | Heptanoat | -H | -C(O)-CH₂-N⁺(CH₃)₃ |
| 1,1-6 | Caprylat | -H | -C(O)-CH₂-N⁺(CH₃)₃ |
| 1,1-6 | Nonanoat | -H | -C(O)-CH₂-N⁺(CH₃)₃ |
| 1,1-6 | Caprat | -H | -C(O)-CH₂-N⁺(CH₃)₃ |
| 1,1-6 | Undecanoat | -H | -C(O)-CH₂-N⁺(CH₃)₃ |
| 1,1-6 | Laurat | -H | -C(O)-CH₂-N⁺(CH₃)₃ |
| 1,1-6 | Tridecanoat | -H | -C(O)-CH₂-N⁺(CH₃)₃ |
| 1,1-6 | Myristat | -H | -C(O)-CH₂-N⁺(CH₃)₃ |
| 1,1-6 | Pentadecanoat | -H | -C(O)-CH₂-N⁺(CH₃)₃ |
| 1,1-6 | Palmitat | -H | -C(O)-CH₂-N⁺(CH₃)₃ |
| 1,1-6 | Heptadecanoat | -H | -C(O)-CH₂-N⁺(CH₃)₃ |
| 1,1-6 | Stearat | -H | -C(O)-CH₂-N⁺(CH₃)₃ |
| 1,1-6 | Isostearat | -H | -C(O)-CH₂-N⁺(CH₃)₃ |
| 1,1-6 | Oleat | -H | -C(O)-CH₂-N⁺(CH₃)₃ |
| 1,1-6 | Ricinoleat | -H | -C(O)-CH₂-N⁺(CH₃)₃ |
| 1,1-6 | Nonadecanoat | -H | -C(O)-CH₂-N⁺(CH₃)₃ |
| 1,1-6 | Arachidat | -H | -C(O)-CH₂-N⁺(CH₃)₃ |
| 1,1-6 | Behenat | -H | -C(O)-CH₂-N⁺(CH₃)₃ |
| 1,1-6 | -C(O)-CH₂-N⁺(CH₃)₃ | -H | Heptanoat |
| 1,1-6 | -C(O)-CH₂-N⁺(CH₃)₃ | -H | Caprylat |
| 1,1-6 | -C(O)-CH₂-N⁺(CH₃)₃ | -H | Nonanoat |
| 1,1-6 | -C(O)-CH₂-N⁺(CH₃)₃ | -H | Caprat |
| 1,1-6 | -C(O)-CH₂-N⁺(CH₃)₃ | -H | Undecanoat |
| 1,1-6 | -C(O)-CH₂-N⁺(CH₃)₃ | -H | Laurat |
| 1,1-6 | -C(O)-CH₂-N⁺(CH₃)₃ | -H | Tridecanoat |
| 1,1-6 | -C(O)-CH₂-N⁺(CH₃)₃ | -H | Myristat |
| 1,1-6 | -C(O)-CH₂-N⁺(CH₃)₃ | -H | Pentadecanoat |
| 1,1-6 | -C(O)-CH₂-N⁺(CH₃)₃ | -H | Palmitat |
| 1,1-6 | -C(O)-CH₂-N⁺(CH₃)₃ | -H | Heptadecanoat |
| 1,1-6 | -C(O)-CH₂-N⁺(CH₃)₃ | -H | Stearat |
| 1,1-6 | -C(O)-CH₂-N⁺(CH₃)₃ | -H | Isostearat |
| 1,1-6 | -C(O)-CH₂-N⁺(CH₃)₃ | -H | Oleat |
| 1,1-6 | -C(O)-CH₂-N⁺(CH₃)₃ | -H | Ricinoleat |
| 1,1-6 | -C(O)-CH₂-N⁺(CH₃)₃ | -H | Nonadecanoat |
| 1,1-6 | -C(O)-CH₂-N⁺(CH₃)₃ | -H | Arachidat |
| 1,1-6 | -C(O)-CH₂-N⁺(CH₃)₃ | -H | Behenat |

Besonders bevorzugte Verbindungen A) sind Betainester gemäß Formel (la),

[R¹(OCH₂CH(OR²)CH₂)ₓ-O-C(O)-CH₂-N⁺(CH₃)₃] A⁻ (la)

in der
- R¹ für -H, -C(O)-CH₂-N⁺(CH₃)₃ oder für Caprylat, Caprat, Laurat, Myristat, Palmitat, Stearat, Isostearat, Oleat, Ricinoleat, Arachidat, Behenat,
- R² für -H,
- x für eine Zahl von 1,1 bis 6, bevorzugt von 2 bis 4 und insbesondere für 3 und
- A⁻ für ein physiologisch akzeptables organisches Anion wie Formiat, Acetat, Propionat, Benzoat, Citrat, Oxalat, Tartat, (Poly)acrylat, Fumarat, Maleat, Lactat, Itaconat, Glykolat, Gluconat, Malat, Mandelat, Tiglat, Succinat, Ascorbat steht.

Beispiele für besonders bevorzugte Verbindungen A) sind Betainester nach Formel (la) gemäß der nachfolgenden Tabelle:

| x | R¹ | R² | A⁻ |
|---|---|---|---|
| 1,1-6; (2-4); (3) | -H | -H | Formiat, Acetat, Propionat, Benzoat, Citrat, Oxalat, Tartat, (Poly)acrylat, Fumarat, Maleat, Lactat, Itaconat, Glykolat, Gluconat, Malat, Mandelat, Tiglat, Succinat, Ascorbat |
| 1,1-6; (2-4); (3) | -C(O)-CH₂-N⁺(CH₃)₃ | -H | |
| 1,1-6; (2-4); (3) | Caprylat | -H | |
| 1,1-6; (2-4); (3) | Caprat | -H | |
| 1,1-6; (2-4); (3) | Laurat | -H | |
| 1,1-6; (2-4); (3) | Myristat | -H | |
| 1,1-6; (2-4); (3) | Palmitat | -H | |
| 1,1-6; (2-4); (3) | Stearat | -H | |
| 1,1-6; (2-4); (3) | Oleat | -H | |
| 1,1-6; (2-4); (3) | Ricinoleat | -H | |
| 1,1-6; (2-4); (3) | Arachidat | -H | |
| 1,1-6; (2-4); (3) | Behenat | -H | |

Ganz besonders bevorzugt sind Betainester nach Formel (la), in der
- R¹ für -H, -C(O)-CH₂-N⁺(CH₃)₃ oder für Oleat,
- R² für -H,
- x für eine Zahl von 1,1 bis 6, besonders bevorzugt von 2 bis 4 und insbesondere für 3 und
- A⁻ für Formiat, Acetat, Propionat, Benzoat, Citrat, Oxalat, Tartat, Lactat oder Malat steht.

Beispiele für ganz besonders bevorzugte Betainester nach Formel (la) sind der nachfolgenden Tabelle zu entnehmen:

| x | R¹ | R² | A⁻ |
|---|---|---|---|
| 1,1-6 | -H | -H | Formiat, Acetat, Propionat, Benzoat, Citrat, Oxalat, Tartat, Lactat, Malat |
| 1,1-6 | -C(O)-CH₂-N⁺(CH₃)₃ | -H | |
| 1,1-6 | Oleat | -H | |
| 2-4 | -H | -H | |
| 2-4 | -C(O)-CH₂-N⁺(CH₃)₃ | -H | |
| 2-4 | Oleat | -H | |
| 3 | -H | -H | |
| 3 | -C(O)-CH₂-N⁺(CH₃)₃ | -H | |
| 3 | Oleat | -H | |

Insbesondere bevorzugte Verbindungen a) oder A) nach Formel (I) oder Formel (la) sind unter der INCI-Bezeichnung Polyglyceryl-3 betainate acetate bekannte Verbindungen.

Entsprechende Handelsprodukte sind beispielsweise von der Firma Produkt unter der Bezeichnung Greenquat^{®} BT erhältlich.

Verbindung(en) a) oder A) wird (werden) in den erfindungsgemäßen Haarbehandlungsmitteln - bezogen auf deren Gesamtgewicht - vorzugsweise in Mengen von 0,01 bis 10 Gew.-% eingesetzt. Die besten Wirkungen wurden beobachtet, wenn die Menge von Verbindung(en) a) oder A) im Bereich von 0,025 bis 7,00 Gew.-% liegt, bezogen auf das Gesamtgewicht des Haarbehandlungsmittels, vorzugsweise von 0,05 bis 5,00 Gew.-%, mehr bevorzugt von 0,075 bis 4,00 Gew.-% und insbesondere von 0,10 bis 3,00 Gew.-%.

Komponente b) in den erfindungsgemäßen Haarbehandlungsmitteln ist mindestens Aktivstoff. Gemäß bevorzugter Ausführungsformen ist der Aktivstoff ausgewählt ist aus der Gruppe bestehend aus einem Xanthin, einem Taurin, einem Vitamin, einem Antischuppenmittel, einem Echinacea-Pflanzenextrakt, einem Mikrobiom-Aktivmittel und einer Kombination davon.

Xanthine, darunter insbesondere Coffein, sowie Taurin und Vitamine üben einen vitalisierenden Effekt auf die Haut aus. Der Echinacea-Extrakt hat eine unterstützende Wirkung bei der Behandlung von Akne. Unter den Mikrobiom-Aktivmittel befinden sich ebenfalls Stoffe, insbesondere das Carnithin, die einen vitalisierenden Effekt auf die Haut ausüben.

Unter einem "Vitamin" soll ein Vitamin, ein Provitamin und eine Vitaminvorstufe verstanden werden. Gemäß bevorzugter Ausführungsformen ist das das Vitamin ausgewählt aus solchen, die den Gruppen A, B, C, E, F und H zugeordnet werden. Insbesondere ist das Vitamin ausgewählt aus der Gruppe bestehend aus 2,4-Dihydroxy-N-(3-hydroxypropyl)-3,3-dimethyl-butyramid, Provitamin B₅), Pantothensäure (Vitamin B₅), Niacin (Vitamin B₃), Nicotinamid (Vitamin B₃), L-Ascorbinsäure (Vitamin C), Thiamin (Vitamin B₁), Riboflavin (Vitamin B₂, Vitamin G), Biotin (Vitamin 87, Vitamin H), Folsäure (Vitamin B₉, Vitamin B₁₁ oder Vitamin M), Vitamin B₆ und Vitamin B12.

Bevorzugt ist das Taurin 2-Aminoethansulfonsäure oder ein Taurinderivat, wobei das Taurinderivat bevorzugt ausgewählt ist aus der Gruppe bestehend aus N-Monomethyl-Taurin oder N,N-Dimethyl-Taurin.

Gemäß einer bevorzugten Ausführungsform der vorliegenden Erfindung ist das Xanthin ausgewählt aus der Gruppe bestehend aus Coffein, Theophyylin, Theobromin und Mischungen davon. Insbesondere handelt es sich bei dem bevorzugt verwendeten Xanthin um Coffein.

Der Echinacea-Pflanzenextrakt wird bevorzugt aus der Pflanze Echinacea purpurea gewonnen.

Der Aktivstoff ist bevorzugt ein Antischuppenmittel. Bevorzugter ist das Antischuppenmittel ein anorganisches Zinksalz. Noch bevorzugter ist das anorganische Zinksalz ein wasserunlösliches anorganisches Zinksalz. Unter "wasserunlöslich" wird im Rahmen der vorliegenden Erfindung ein Salz verstanden, dass sich mit einer Masse von weniger als 1 g, bevorzugt weniger als 100 mg, des Salzes bei 20°C in Wasser löst.

Ein Haarbehandlungsmittel nach einer der Aussagen 31 bis 32, wobei das anorganische Zinksalz ausgewählt ist aus der Gruppe bestehend aus Zinkpyrithion, Zinkaluminat, Zinkcarbonat, Zinkoxid, Calamine, Zinkphosphate, Zinkorthophosphat, Zinkpyrophosphat, Zinkselenid, Zinksulfid, Zinksilikat, Zinkorhtosilikat, Zinkmetalsilikat, Zinkborat, Zinkhydroxid und Zinkhydroxysulfat, insbesondere Zinkoxid oder Zinkcarbonat ist.

Ferner kann das Antischuppenmittel ausgewählt sein aus der Gruppe bestehend aus Piroctone Olamine, Climbazol, Ketoconazole, Salicylsäure, Schwefel, Selensulfid, Teerpräparate, Undecensäurederivate, Klettenwurzelextrakte, Pappelextrakte, Brennesselextrakte, Walnussschalenextrakte, Birkenextrakte, Weidenrindenextrakte, Rosmarinextrakte, Arnikaextrakte und Propandiol Caprylate, wobei das Antischuppenmittel insbesondere Propandiol Caprylate ist.

Ein weiterer Aktivstoff ist bevorzugt ein Mikrobiom-Aktivmittel. Unter einem "Mikrobiom-Aktivmittel" wird ein Stoff verstanden, der aus einem Mikrobiom, insbesondere aus gramnegativen Bakterien, gewonnen wird.

Bevorzugt ist das Mikrobiom-Aktivmittel Carnitin, ein Carnitin-Derivat oder ein Carnitinsalz. Bevorzugter ist das Mikrobiom-Aktivmittel ausgewählt aus der Gruppe bestehend aus L-Carnitin, Acetyl-L-Carnitin, L-Carnitin-Fumarat, L-Carnitin-Citrat, Lauroyl-L-Carnitin und L-Carnitin-Tartrat, insbesondere ist das Mikrobiom-Aktivmittel L-Carnitin-Tartrat.

Das bevorzugt verwendete Vitamin, Provitamin oder Vitaminvorstufe ist bevorzugt in einer Menge von 0,001 bis 10 Gew.-%, bevorzugter von 0,01 bis 2 Gew.-% und noch bevorzugter von 0,1 bis 1 Gew.-% bezogen auf das Gesamtgewicht des Haarbehandlungsmittels in dem Haarbehandlungsmittel enthalten.

Wenn der Aktivstoff ein Antischuppenmittel ist, ist das Antischuppenmittel bevorzugt in einer Menge von 0,01 bis 5 Gew.-%, bevorzugter 0,02 bis 4 Gew.-%, noch bevorzugter von 0,05 bis 3 Gew.-% und am meisten bevorzugt von 0,1 bis 1 Gew.-% bezogen auf das Gesamtgewicht des Haarbehandlungsmittels in dem Haarbehandlungsmittel enthalten.

Wenn der Aktivstoff ein Mikrobiom-Aktivmittel ist, ist dieses bevorzugt in einer Menge von 0,001 bis 10 Gew.-%, bevorzugter von 0,01 bis 2 Gew.-% und noch bevorzugter von 0,1 bis 1 Gew.-% bezogen auf das Gesamtgewicht des Haarbehandlungsmittels in dem Haarbehandlungsmittel enthalten.

Das Taurin oder das Taurin-Derivat ist bevorzugt in einer Menge von 0,001 bis 10 Gew.-%, bevorzugter von 0,01 bis 2 Gew.-% und noch bevorzugter von 0,1 bis 1 Gew.-% bezogen auf das Gesamtgewicht des Haarbehandlungsmittels in dem Haarbehandlungsmittel enthalten.

Das Xanthin, insbesondere das Coffein, ist bevorzugt in einer Menge von 0,001 bis 10 Gew.-%, bevorzugter von 0,01 bis 2 Gew.-% und noch bevorzugter von 0,1 bis 1 Gew.-% bezogen auf das Gesamtgewicht des Haarbehandlungsmittels in dem Haarbehandlungsmittel enthalten.

Der Echinacea-Pflanzenextrakt ist bevorzugt in einer Menge von 0,001 bis 10 Gew.-%, bevorzugter von 0,01 bis 2 Gew.-% und noch bevorzugter von 0,1 bis 1 Gew.-% bezogen auf das Gesamtgewicht des Haarbehandlungsmittels in dem Haarbehandlungsmittel enthalten.

Ferner kann das Haarbehandlungsmittel einen natürlichen Verdicker enthalten. Unter einem "natürlichen Verdicker" wird im Rahmen der vorliegenden Erfindung ein Verdicker verstanden, der aus natürlichen Quellen stammt. Im Gegensatz dazu ist ein nicht-natürlicher Verdicker ein Stoff, der aus petrochemischen Quellen synthetisch hergestellt wird. Unter einen "natürlichen Verdicker" sollen auch Verdicker fallen, die nicht unter die "nicht-natürlichen" Verdicker fallen, jedoch chemisch modifiziert sind.

Gemäß einer bevorzugten Ausführungsform ist der natürliche Verdicker ausgewählt aus der Gruppe bestehend aus einem natürlichen Polymer, einem modifizierten Tonmineral, einem unmodifizierten Tonmineral, einem modifizierten Silikat und einem unmodifizierten Silikat.

Bevorzugt ist das natürliche Polymer ein modifiziertes natürliches Polymer. Grundlage des modifizierten natürlichen Polymers ist ein natürliches Polymer, das modifiziert wird.

Gemäß einer bevorzugten Ausführungsform ist der natürliche Verdicker ein modifiziertes oder unmodifiziertes, ionisches oder nicht-ionisches Polysaccharid. Bevorzugter ist der natürliche Verdicker ausgewählt aus der Gruppe bestehend aus einem Xanthan, Agar, einem Chitosan, einer Stärke und einer Kombination davon.

Insbesondere ist der natürliche Verdicker ausgewählt aus der Gruppe bestehend aus den unter folgenden INCI-Bezeichnungen bekannten polymeren Verdickungsmitteln: Agar, Agarose, Alcaligenes Polysaccharides, Algin, Alginic Acid, Ammonium Alginate, Amylopectin, Ascorbyl Methylsilanol Pectinate, Astragalus Gummifer Gum, Attapulgite, Avena Sativa (Oat) Kernel Flour, Butoxy Chitosan, Caesalpinia Spinosa Gum, Calcium Alginate, Calcium Carboxymethyl Cellulose, Calcium Carrageenan, Calcium Starch Octenylsuccinate, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Gelatin, Gellan Gum, Glyceryl Alginate, Glycine Soja (Soybean) Flour, Guar Hydroxypropyltrimonium Chloride, Hydrogenated Potato Starch, Hydroxybutyl Methylcellulose, Hydroxyethylcellulose, Hydroxyethyl Chitosan, Hydroxyethyl Ethylcellulose, Hydroxypropylcellulose, Hydroxypropyl Chitosan, Hydroxypropyl Guar, Hydroxypropyl Methylcellulose, Hydroxypropyl Methylcellulose Stearoxy Ether, Macrocystis Pyrifera (Kelp), Magnesium Alginate, Methylcellulose, Methyl Ethylcellulose, Methyl Hydroxyethylcellulose, Microcrystalline Cellulose, Natto Gum, Nonoxynyl Hydroxyethylcellulose, Pectin, PEG-4 Rapeseedamide, Potassium Alginate, Potassium Carrageenan, Potato Starch Modified, Propylene Glycol Alginate, Rhizobian Gum, Sclerotium Gum, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Starch Octenylsuccinate, Sodium Stearoxy PG-Hydroxyethylcellulose Sulfonate, Starch/Acrylates/Acrylamide Copolymer, Starch Hydroxypropyltrimonium Chloride, Sterculia Urens Gum, Tamarindus Indica Seed Gum, Tapioca Starch, TEA-Alginate, Triticum Vulgare (Wheat) Starch, Welan Gum, Xanthan, Xanthan Gum, Yeast Beta-Glucan, Yeast Polysaccharides und Zea Mays (Corn) Starch.

Ganz besonders bevorzugt ist der natürliche Verdicker ausgewählt aus Xanthan Gum oder Caesalpinia Spinosa Gum.

Alternativ oder zusätzlich zu den natürlichen Verdickern auf Basis eines polymeren Bestandteils kann das Tonmineral ausgewählt sein aus der Gruppe bestehend aus den folgenden Tonmineralien: Bentonit, Hectorit, Montmorillonit und Kombinationen davon.

Insbesondere ist der natürliche Verdicker ausgewählt aus der Gruppe bestehend aus den unter folgenden INCI-Bezeichnungen bekannten Verdickern: Bentonite, Dihydrogenated Tallow Benzylmonium Hectorite, Hectorite, Hydrated Silica, Lithium Magnesium Silicate, Lithium Magnesium Sodium Silicate, Magnesium Aluminum Silicate, Magnesium Silicate, Magnesium Trisilicate, Montmorillonite, Moroccan Lava Clay, PVP Montmorillonite, Sodium Magnesium Fluorosilicate, Sodium Silicoaluminate, Tromethamine Magnesium Aluminum Silicate, und Kombinationen davon.

Ferner kann das Haarbehandlungsmitteln ein Öl umfassen. Das Öl soll einen Schmelzpunkt bei Normaldruck (1013 mbar) von weniger als 25°C aufweisen. Für den Fall, dass ein Gemisch von mehreren Ölen eingesetzt wird, soll bevorzugt das Gemisch der Öle einen Schmelzpunkt bei Normaldruck von weniger als 25°C aufweisen.

Bevorzugt ist das Öl oder jedes Öl ausgewählt aus einem natürlichen Öl, einem synthetischen Öl, einem Esteröl oder einem aminofunktionellen Silikon.

Unter einem Esteröl kann auch ein Glycerinester verstanden werden. In jedem Fall unterscheiden sich die Komponente a) und das Esteröl voneinander. Wenn das Esteröl eine Glycerineinheit aufweist, liegt die Zahl der Glycerineinheiten bei 1 und somit unter 1,1, wie bei den Verbindungen der Komponente a).

Unter einem natürlichen Öl soll ein Öl verstanden werden, das aus einer natürlichen Quelle stammt und nicht aus synthetischen Einheiten chemisch erzeugt wird. Das natürliche Öl kann jedoch ein Öl sein, das chemisch modifiziert wird. Das natürliche Öl ist bevorzugt ein pflanzliches Öl. Das pflanzliche Öl wird aus einer Pflanze gewonnen. Beispielsweise wird dabei pflanzliches Material durch einen mechanischen Vorgang bearbeitet, so dass das Öl gewonnen wird. Das pflanzliche Öl ist bevorzugt ein kaltgepresstes Öl.

Das pflanzliche Öl ist beispielsweise ausgewählt aus der Gruppe bestehend aus Amaranthsamenöl, Aprikosenkernöl, Arganöl, Avocadoöl, Babassuöl, Baumwollsaatöl, Borretschsamenöl, Camelinaöl, Chiaöl, Distelöl, Erdnussöl, Granatapfelkernöl, Grapefruitsamenöl, Hanföl, Haselnussöl, Holundersamenöl, Johannisbeersamenöl, Jojobaöl, Kakaobutter, Krambesamenöl, Kürbiskernöl, Leinöl, Leinsamenöl, Macadamianussöl, Maiskeimöl, Mandelöl, Marulaöl, Mohnöl, Moringaöl, Nachtkerzenöl, Olivenöl, Palmöl, Palmkernöl, Perillaöl, Pfirsichkernöl, Rapsöl, Reisöl, Reiskleieöl, Rizinusöl, Sacha Inchi-Öl, Sanddornfruchtfleischöl, Sanddornkernöl, Sesamöl, Sojaöl, Sonnenblumenöl, Traubenkernöl, Walnussöl, Weizenkeimöl, Wildrosenöl und den flüssigen Anteilen des Kokosöls, wobei Arganöl, Avocadoöl und Jojobaöl besonders bevorzugt sind.

Gemäß einer weiteren bevorzugten Ausführungsform ist das Öl ein synthetisches Öl, das bevorzugter ausgewählt ist aus einem verzweigten oder unverzweigten Alkan mit 10 bis 30 Kohlenstoffatomen, insbesondere ausgewählt ist aus der Gruppe bestehend aus Isodecan, Isoundecan, Isododecan, Isotridecan, Isotetradecan, C12-17-Alkan und Gemischen davon.

Das Esteröl wird bevorzugt aus mindestens einer linearen oder verzweigten, gesättigten oder ungesättigten Mono-, Di- oder Tricarbonsäure mit 2 bis 30 Kohlenstoffatomen, welche gegebenenfalls eine oder mehrere Hydroxylgruppen enthalten kann, und mindestens einem linearen oder verzweigten, gesättigten oder ungesättigten Alkohol mit 1 bis 30 Kohlenstoffatomen gebildet. Beispielsweise ist das Esteröl ausgewählt aus der Gruppe bestehend aus: Isopropylmyristat, Isopropylpalmitat, Isopropyllaurat, Isopropyllanolinat, Isopropylricinoleat, Isopropylstearat, Isononylnonanoat, Alkylbenzoat, Dicaprylylcarbonat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylpalmitat, 2-Ethylhexylstearat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Cetyloleat, Oleyloleat, Oleylerucat, Erucyloleat, Erucylerucat, Kokosfettalkohol-caprinat, Kokosfettalkohol-caprylat und Mischungen davon.

Das Öl kann ferner ein aminofunktionelles Silikon sein, wobei dieses ein aminofunktionalisiertes Silikonpolymer (a1) mit mindestens einer sekundären Aminogruppe ist. Das aminofunktionalisierte Silikonpolymer (a1) umfasst mindestens eine Struktureinheit der Formel (Si-Amino), 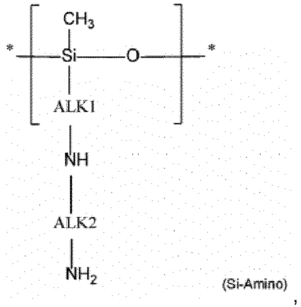 wobei ALK1 und ALK2 unabhängig voneinander für eine lineare oder verzweigte, zweiwertige C1 - C20-Alkylengruppe stehen.

Bevorzugt umfasst das aminofunktionalisierte Silikonpolymer (a1) Struktureinheiten der Formel (Si-I) und der Formel (Si-II)

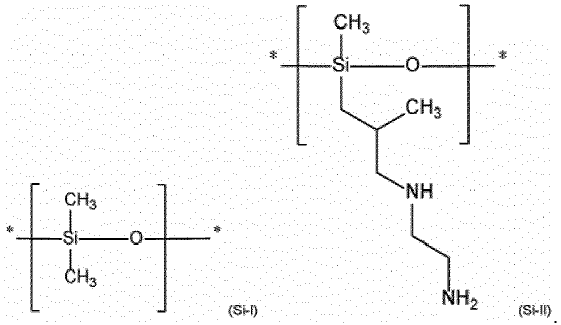

Bevorzugter ist das aminofunktionelle Silikonpolymer (a1) ein aminofunktionelles Silikonpolymer der Formel (SiIII), 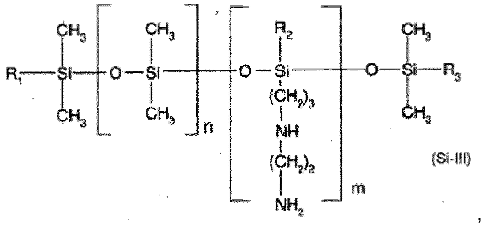 wobei
- m und n bedeuten Zahlen, die so gewählt sind, dass die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet.

Die hierin offenbarten Haarbehandlungsmittel können als Öl bevorzugt umfassen:
- mindestens einen Mono-, Di- oder Triester von Glycerin und eine Fettsäure mit 8 bis 30 Kohlenstoffatomen, und/oder
- mindestens einen Mono- oder Diester von Ethylenglykol und eine Fettsäure mit 8 bis 30 Kohlenstoffatomen.

Die C8-C30-Komponente der Fettsäure kann gesättigt oder ungesättigt, verzweigt oder unverzweigt sein. Geeignete Beispiele sind unter anderem Glycerylstearat und Glycoldistearat.

Vorzugsweise umfasst das erfindungsgemäße Haarbehandlungsmittel nur eines der obigen Beispiele. Alternativ umfasst das Haarbehandlungsmittel eine Mischung aus zwei der obigen Beispiele. Eine andere Alternative besteht darin, dass das Haarbehandlungsmittel drei oder mehr der obigen Beispiele umfasst.

Gemäß einer bevorzugten Kombination ist das Öl eine Kombination aus einem natürlichen Öl und einem Esteröl und einem aminofunktionellen Silikon, oder eine Kombination aus einem natürlichen Öl und einem Esteröl, oder eine Kombination aus einem natürlichen Öl und einem aminofunktionellen Silikon. Dabei liegt bevorzugt das Gewichtsverhältnis von natürlichem Öl zu Esteröl zwischen 5:1 und 1:1, bevorzugt 4:1 und 2:1; bzw. das Gewichtsverhältnis von natürlichem Öl zu aminofunktionellem Öl zwischen 4:1 und 1:2, bevorzugt 3:1 und 1:1.

Die Gesamtmenge an Öl kann im Bereich von 0,001 bis 10 Gew.-% liegen, bezogen auf das Gesamtgewicht der Zusammensetzung. Die besten Wirkungen wurden beobachtet, wenn die Menge an Öl im Bereich von 0,01 bis 5 Gew.-%, bezogen auf das Gesamtgewicht der Zusammensetzung, vorzugsweise im Bereich von 0,01 bis 3 Gew.-%, mehr bevorzugt im Bereich von 0,1 bis 1 Gew.-%, und insbesondere im Bereich von 0,15 bis 0,95 Gew.-% liegt.

Gemäß bevorzugter Ausführungsformen umfasst das Haarbehandlungsmittel ferner ein mehrere Proteolipide. Bevorzugt weist das oder jedes Proteolipid die Formel (II) oder (III) auf,

R'-X-R" (II) [R'-X-R"]⁺ Z⁻ (III)

wobei
- R' eine geradkettige oder verzweigte, gesättigte oder ungesättigte funktionelle Kohlenwasserstoffgruppe mit 8 bis 24 Kohlenstoffatomen darstellt (einschließlich Gemischen solcher R'-Gruppen, wie sie beispielsweise in Naturstoffen wie Kokosnussöl enthalten sind).
- R" ein Protein, ein Peptid oder ein Proteinhydrolysat darstellt,
- Z⁻ ein physiologisch verträgliches Anion darstellt
- X -C(O)O- oder -N⁺(R^{III}₂)-R^{IV}- oder -N(R^{III})R^{IV}- oder -C(O)-N(R^{V})R^{VI} darstellt,
- R^{III} -(CH₂)ₓ-CH₃ darstellt, wobei x 0-22 ist, und
- R^{IV} -CH₂-CH(OH)-CH₂- oder -(CH₂)ₓ- darstellt, gegebenenfalls substituiert mit einer oder mehreren -OH-Gruppen, wobei x = 0-22 ist;
- R^{v} und R^{VI} unabhängig voneinander -H oder -(CH₂)ₓ-CH₃ darstellen, wobei x = 0-22 ist;
mit der Maßgabe, dass R" Keratin oder ein Keratinhydrolysat darstellt, wenn X -C(O)O- darstellt.

Wenn X -C(O)O- oder -N(R^{III})R^{IV}- oder -C(O)-N(R^{V})R^{VI} darstellt, ist die Verbindung ungeladen und es gilt Formel (II). Wenn X -N⁺(R^{III}₂)-R^{IV}- darstellt, d.h., der Stickstoff quaternisiert ist, ist ein Gegenion vorhanden und es gilt die Formel (III). Das Gegenion ist jedes geeignete physiologisch verträgliche Anion wie ein Halogenid. Ein bevorzugtes Kation ist Chlorid.

Geeigneter ist, dass das oder jedes Proteolipid die Formel (IVA) aufweist: 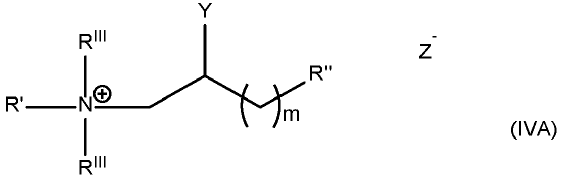 wobei
- R', R^{III}, R" und Z wie zuvor definiert sind,
- m = 1 bis 10, und
- Y für -H oder -OH steht.

R" kann ein Protein sein. Alternativ kann R" ein Peptid sein. R" kann auch ein Proteinhydrolysat sein. Vorzugsweise ist R" ein Proteinhydrolysat. Ein solches Proteinhydrolysat kann auf Keratin basieren, d.h. auf einem Keratinhydrolysat. Alternativ kann R" ein Hydrolysat auf pflanzlicher Basis sein.

Bevorzugte Beispiele für Proteolipide sind unter anderem:
- Hydrolysiertes Steardimoniumhydroxypropyl-Keratin,
- Hydrolysiertes Steardimoniumhydroxypropyl-Kollagen,
- Hydrolysiertes Steardimoniumhydroxypropyl-Seidenprotein,
- Hydrolysiertes Steardimoniumhydroxypropyl-Kaschmirprotein,
- Hydrolysiertes Steardimoniumhydroxypropyl-Reisprotein,
- Hydrolysiertes Steardimoniumhydroxypropyl-Weizenprotein,
- Hydrolysiertes Steardimoniumhydroxypropyl-Sojaprotein,
- Hydrolysiertes Steardimoniumhydroxypropyl-Erbsenprotein,
- Hydrolysiertes Steardimoniumhydroxypropyl-Baumwollsamenprotein,
- Hydrolysiertes Steardimoniumhydroxypropyl-Hanfsamenprotein,
- Hydrolysiertes Cocodimoniumhydroxypropyl-Keratin,
- Hydrolysiertes Cocodimoniumhydroxypropyl-Kollagen,
- Hydrolysiertes Cocodimoniumhydroxypropyl-Seidenprotein,
- Hydrolysiertes Cocodimoniumhydroxypropyl-Kaschmirprotein,
- Hydrolysiertes Cocodimoniumhydroxypropyl-Reisprotein,
- Hydrolysiertes Cocodimoniumhydroxypropyl-Sojaprotein,
- Hydrolysiertes Cocodimoniumhydroxypropyl-Weizenprotein,
- Hydrolysiertes Cocodimoniumhydroxypropyl-Erbsenprotein
- Hydrolysiertes Cocodimoniumhydroxypropyl-Baumwollsamenprotein,
- Hydrolysiertes Cocodimoniumhydroxypropyl-Hanfsamenprotein,

Dem Fachmann ist bekannt, dass kosmetische Produkte häufig mindestens ein Tensid enthalten, das aus kationischen, anionischen, amphoteren, zwitterionischen und/oder nichtionischen Tensiden ausgewählt ist.

Vorzugsweise umfasst das hier definierte Haarbehandlungsmittel ein oder mehrere Tenside, ausgewählt aus:
(1) mindestens einem kationischen Tensid;
(2) mindestens einem anionischen Tensid;
(3) mindestens einem amphoteren und/oder zwitterionischen Tensid; und/oder
(4) mindestens einem nichtionischen Tensid.

Vorzugsweise wird das Haarbehandlungsmittel der vorliegenden Erfindung als Haarconditioner oder Treatment konfektioniert, wobei das Haarbehandlungsmittel vorzugsweise mindestens ein kationisches Tensid und gegebenenfalls mindestens ein anionisches, amphoteres, zwitterionisches oder nichtionisches Tensid umfasst.

Kationische Tenside tragen eine positive Ladung in ihrem hydrophilen Teil. Diese positive Ladung bewirkt, dass sich die Tensidmoleküle an die negativ geladene Haut- und Haaroberfläche binden. Auf diese Weise neutralisieren sie die Ladung, verhindern das Fliegen der Haare, wirken glättend, erhöhen den Haarglanz und verbessern die Nasskämmbarkeit. In erster Linie werden sie in Conditionern, Haarconditionern und Treatments verwendet, seltener in Shampoos. Darüber hinaus wirken kationische Tenside aufgrund ihrer bakteriziden Wirkung, d.h. ihrer bakterienhemmenden Wirkung, in kosmetischen Produkten gleichermaßen konservierend.

Grundsätzlich eignen sich alle kationischen Tenside, die zur Verwendung am menschlichen Körper geeignet sind, als kationische Tenside in erfindungsgemäßen Zusammensetzungen. Diese sind durch mindestens eine wasserlöslich machende kationische Gruppe, wie beispielsweise eine quaternäre Ammoniumgruppe, oder durch mindestens eine wasserlöslich machende kationisierbare Gruppe wie beispielsweise eine Amingruppe und darüber hinaus mindestens eine lipophile Alkylgruppe mit etwa 6 bis 30 Kohlenstoffatome oder auch durch mindestens eine Imidazolgruppe oder mindestens eine Imidazylalkylgruppe gekennzeichnet.

Im Allgemeinen werden kationische Tenside gemäß ihren strukturellen Eigenschaften in Gruppen eingeteilt. Für den Einsatz in den hier definierten Haarbehandlungsmitteln eignen sich insbesondere kationische Tenside a) aus mindestens einer der Gruppen von Alkylquats, Esterquats, quaternären Imidazolinen, Amidoaminen und/oder kationisierten Amidoaminen.

Wenn vorhanden, und unabhängig von der Form des Haarbehandlungsmittels, ist das kationische Tensid (1) mindestens eine Verbindung, ausgewählt aus der Gruppe:
a. Alkylquats (quaternären Ammoniumverbindungen),
b. Esterquats,
c. quarternärem Imidazolin,
d. Amidoaminen und/oder kationisierten Amidoaminen, und
e. Gemischen davon.

Besonders bevorzugte quarternäre Ammoniumverbindungen (1)a. weisen mindestens ein C8-C24-Alkylradikal auf und sind Ammoniumhalogenide, insbesondere Chloride oder Ammoniumalkylsulfate, wie Methosulfate oder Ethosulfate, beispielsweise C8-C24-Alkyltrimethylammoniumchloride, C8-C24-Dialkyldimethylammoniumchloride und C8-C24-Trialkylmethylammoniumchloride Chloride, z.B. Behentrimoniumchlorid, Cetrimoniumchlorid, Steartrimoniumchlorid, Distearyldimethylammoniumchlorid, Lauryldimethylammoniumchlorid, Lauryldimethylbenzylammoniumchlorid und Tricetylmethylammoniumchlorid. Die Alkylketten der oben genannten Tenside weisen vorzugsweise 8 bis 24 Kohlenstoffatome auf. Vorzugsweise umfasst die kationische Tensidkomponente (1)a. Cetrimoniumchlorid und/oder Behentrimoniumchlorid.

Bei Esterquats (1)b. handelt es sich um kationische Tenside, die sowohl mindestens eine Esterfunktion als auch mindestens eine quaternäre Ammoniumgruppe als Strukturelement und ferner mindestens ein C8-C24-Alkylradikal oder ein C8-C24-Acylradikal enthalten. Esterquats können die folgende Formel aufweisen: 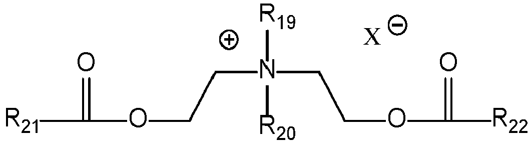 wobei
- R₁₉ und R₂₀ unabhängig voneinander eine C1-C6-Alkylgruppe oder eine C2-C6-Hydroxyalkylgruppe darstellen,
- R₂₁, R₂₂ unabhängig voneinander eine C7-C27-Alkylgruppe, vorzugsweise eine C10-C22-Alkylgruppe, darstellen und
- X⁻ ein physiologisch verträgliches Anion ist,

Bevorzugte Esterquats (1)b. sind quaternierte Estersalze von Fettsäuren mit Triethanolamin, quaternierte Estersalze von Fettsäuren mit Diethanolalkylaminen und quaternierte Estersalze von Fettsäuren mit 1,2-Dihydroxypropyldialkylaminen. Solche Produkte werden beispielsweise unter den Handelsbezeichnungen Stepantex^{®}, Dehyquart^{®} und Armocare^{®} vertrieben. N,N-Bis(2-palmitoyloxyethyl)dimethylammonium chlorid, Distearoylethyldimoniummethosulphate und Distearoylethylhydroxyethylmoniummethosulfate sind bevorzugte Beispiele für solche Esterquats, insbesondere Distearoylethylhydroxyethylmoniummethosulfat. Ein weiteres bevorzugtes Esterquat ist Behenoyl-PG-Trimoniumchlorid.

Wenn vorhanden, kann das quaternäre Imidazolin (1)c. die folgende Formel aufweisen: 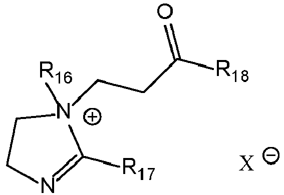 wobei
- R₁₆ eine C1-C6-Alkylgruppe darstellt,
- R₁₇, R₁₈ unabhängig voneinander eine C7-C27-Alkylgruppe darstellen, vorzugsweise eine C10-C22-Alkylgruppe, und
- X⁻ ist ein physiologisch verträgliches Anion ist. Geeignete solche Verbindungen sind unter anderem Quaternium-87 (erhältlich von Evonik als Varisoft W 575 PG).

Geeignete Imidazoliumverbindungen (1)c. sind unter den INCI-Bezeichnungen Quaternium-27, Quaternium-83, Quaternium-87 und Quaternium-91 bekannt. Eine bevorzugte Verbindung ist Quaternium-87.

Alkylamidoamine (1)d. werden üblicherweise durch Amidierung von natürlichen oder synthetischen C₈-C₂₄-Fettsäuren mit Di-(C₁-C₃)-alkylaminoaminen hergestellt.

Geeignete Amine (1)d. können die folgende Formel aufweisen:

NR₂₃R₂₄R₂₅

wobei
- R₂₃, R₂₄ unabhängig voneinander eine C₁-C₆-Alkylgruppe, eine C₂-C₆-Alkenylgruppe oder eine C₂-C₆-Hydroxyalkylgruppe darstellen, und
- R₂₅ eine C₈-C₂₈-Alkylgruppe, vorzugsweise eine C₁₀-C₂₂-Alkylgruppe, darstellt.

Die kationischen Tenside der Formel NR₂₃R₂₄R₂₅ sind Aminderivate, sogenannte Pseudoquats. Die organischen Reste R₂₃, R₂₄ und R₂₅ sind direkt an das Stickstoffatom gebunden. Im sauren pH-Bereich werden diese kationisiert, d.h., das Stickstoffatom wird dann protoniert. Alle physiologisch verträglichen Gegenionen eignen sich als Gegenionen. Stearamidopropyldimethylamin, Behenamidopropyldimethylamin und Brassicamidopropyldimethylamin sind als diese kationischen Tenside besonders bevorzugt.

Das kationische Tensid (1) kann eine Mischung aus einem oder mehreren Alkylquats (1)a. und einem oder mehreren Amidoaminen (1)d. umfassen oder daraus bestehen.

Vorzugsweise wird das Haarbehandlungsmittel der vorliegenden Erfindung als Haarshampoo verwendet, so dass das Haarbehandlungsmittel hauptsächlich ein anionisches Tensid und gegebenenfalls mindestens ein kationisches, amphoteres, zwitterionisches oder nichtionisches Tensid umfasst. Vorzugsweise umfasst ein solches Shampoo mindestens ein anionisches Tensid, mindestens ein amphoteres oder zwitterionisches Tensid und gegebenenfalls mindestens ein nichtionisches Tensid.

Unabhängig von der hier definierten Form des Haarbehandlungsmittels sind Beispiele für die anionischen, amphoteren, zwitterionischen und nichtionischen Tenside wie folgt.

Als anionische Tenside in den erfindungsgemäßen Zusammensetzungen eignen sich alle für die Verwendung am menschlichen Körper geeigneten anionischen oberflächenaktiven Stoffe. Diese sind durch eine wasserlöslich machende, anionische Gruppe wie beispielsweise eine Carboxylat-, Sulfat-, Sulfonat- oder Phosphatgruppe und eine lipophile Alkylgruppe mit 8 bis 30 C-Atomen gekennzeichnet. Zusätzlich können im Molekül Glykol- oder Polyglykolethergruppen, Ester-, Ether- und Amidgruppen sowie Hydroxylgruppen enthalten sein. Beispiele für geeignete anionische Tenside sind lineare und verzweigte Fettsäuren mit 8 bis 30 C-Atomen (Seifen), Alkylethercarbonsäuren, Carboxylattenside wie Acylglycinate, Acylsarcosinate, Acyllactylate, Acylglutamate, Acylaspartate, Acyltaurate, Acylisethionate, Sulfosuccinate sowie die entsprechenden versalzten Formen der vorgenannten Tenside verstanden, wobei die Acylgruppen 10 bis 30 Kohlenstoffatome umfassen, lineare Alkansulfonate, lineare alpha-Olefinsulfonate, Alkylsulfate und Alkylethersulfate sowie Alkyl- und/oder Alkenylphosphate. Bevorzugte anionische Tenside sind Alkylsulfate, Alkylethersulfate und Alkylethercarbonsäuren mit jeweils 10 bis 18 C-Atomen, bevorzugt 12 bis 14 C-Atomen, in der Alkylgruppe und bis zu 12 Glykolethergruppen, bevorzugt 2 bis 6 Glykolethergruppen, im Molekül.

Das positiv geladene Gegenion kann aus jedem kosmetisch geeigneten Gegenion ausgewählt werden, wie der Fachmann verstehen wird. Geeignete Gegenionen sind Alkalimetalle (vorzugsweise Natrium oder Kalium) und Ammonium. Beispiele für solche Tenside sind die Verbindungen mit den INCI-Bezeichnungen Natriumlaurethsulfat, Ammoniumlaurylsulfat, Natriumlaurylsulfat, Natriummyrethsulfat oder Natriumlaurethcarboxylat. Bevorzugte anionische Tenside sind Natriumlaurethsulfat, Ammoniumlaurylsulfat oder eine Mischung davon.

Weiterhin bevorzugt sind anionische Carboxylattenside aus den zuvor genannten Gruppen, sowie deren entsprechende versalzte Formen, in denen die Acylgruppen 10 bis 24 Kohlenstoffatome und insbesondere 10 bis 18 Kohlenstoffatome umfassen.

Beispiele für besonders geeignete anionische Carboxylattenside sind die Alkali-, Erdalkali- und/oder Alkanolaminsalze von Cocoyl Glycinate, Cocoyl Sarcosinate, Lauroyl Sarcosinate, Myristoyl Sarcosinate, Oleyl Sarcosinate, Lauroyl Lactylate, Stearoyl Lactylate, Cocoyl Glutamate, Lauroyl Glutamate, Stearoyl Glutamate, Lauroyl Aspartate, Palmitoyl Aspartate, (C₁-C₄-Alkyl) Cocoyl Taurate, (C₁-C₄-Alkyl) Lauroyl Taurate, (C₁-C₄-Alkyl) Oleyl Taurate, (C₁-C₄-Alkyl) Cocoyl Isethionate, (C₁-C₄-Alkyl) Lauroyl Isethionate, Lauryl Sulfosuccinate, Lauryl Sulfoacetate und/oder beliebige Mischungen davon.

Als zwitterionische Tenside werden solche oberflächenaktiven Verbindungen bezeichnet, die im Molekül mindestens eine quaternäre Ammoniumgruppe und mindestens eine Carboxylat-, Sulfonat- oder Sulfatgruppe tragen. Besonders geeignete zwitterionische Tenside sind die sogenannten Betaine wie die N-Alkyl-N,N-dimethylammoniumglycinate, beispielsweise Cocoalkyldimethylammoniumglycinat, N-Acyl-aminopropyl-N,N-dimethylammoniumglycinate, beispielsweise Cocoacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie Cocoacylaminoethylhydroxyethylcarboxymethylglycinat. Ein bevorzugtes zwitterionisches Tensid ist das unter der INCI-Bezeichnung Cocoamidopropylbetaine bekannte Fettsäureamidderivat.

Unter amphoteren Tensiden werden solche oberflächenaktiven Verbindungen verstanden, die zusätzlich zu einer C₈-C₂₄-Alkyl- oder -Acylgruppe im Molekül mindestens eine freie Aminogruppe und mindestens eine -COOH- oder-SOsH-Gruppe enthalten und zur Ausbildung innerer Salze in der Lage sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 24 C-Atomen in der Alkylgruppe. Besonders bevorzugte amphotere Tenside sind N-Cocoalkylaminopropionat, Cocoacylaminoethylaminopropionat und C₁₂-C₁₈-Acylsarcosin.

Nichtionische Tenside können ein hydrophiles Ende aufweisen, das ladungsneutral sein kann, wie etwa ein Ethoxylat, Glykosid oder Polyol; derartige Tenside sind möglicherweise nicht wasserhärteempfindlich. Geeignete nichtionische Tenside, wie hierin in Betracht gezogen, sind unter anderem die Folgenden:
- Anlagerungsprodukte von etwa 4 bis etwa 30 Mol Ethylenoxid und/oder etwa 0 bis etwa 5 Mol Propylenoxid mit linearen Fettalkoholen mit etwa 8 bis etwa 22 C-Atomen oder Fettsäuren mit etwa 12 bis etwa 22 C-Atomen oder Alkylphenole mit etwa 8 bis etwa 15 C-Atomen in der Alkylgruppe,
- Anlagerungsprodukte von Ethylenoxid und Polyglycerin mit Methylglukosidfettsäureestern, Fettsäurealkanolamiden und Fettsäureglukamiden,
- C8-C30-Fettsäuremono- und -diester von Additionsprodukten von etwa 1 bis etwa 30 Mol Ethylenoxid oder Glycerin, beispielsweise solche mit den INCI-Bezeichnungen PEG(1-10)-Glycerylcocoat, insbesondere PEG-7-Glycerylcocoat.
- Aminoxide, zum Beispiel mindestens eine Verbindung mit der allgemeinen Formel (I) oder (II): 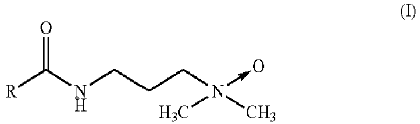 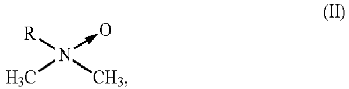
   ∘ wobei R jeweils einen linearen oder verzweigten, gesättigten oder einfach oder mehrfach ungesättigten Alkyl- oder Alkenylrest darstellt, der etwa 6 bis etwa 24 Kohlenstoffatome enthält, beispielsweise etwa 8 bis etwa 18 Kohlenstoffatome enthält.
   ∘ Geeignete Tenside sind solche mit den vorgenannten Formeln (I) oder (II) mit den INCI-Bezeichnungen Cocaminoxid, Lauraminoxid und/oder Cocamidopropylaminoxid, die von einer Reihe verschiedener Anbieter im Handel erhältlich sind.
- Sorbitanfettsäureester und Anlagerungeprodukte von Ethylenoxid mit Sorbitanfettsäureestern wie beispielsweise Polysorbate,
- Fettsäurealkanolamide mit der nachfolgenden allgemeinen Formel: 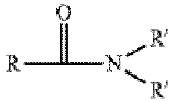
   ∘ wobei R geeigneterweise einen linearen oder verzweigten, gesättigten oder ungesättigten Alkyl- oder Alkenylrest darstellt, der etwa 8 bis etwa 24 Kohlenstoffatome enthält, und die Reste R' Wasserstoff oder die Gruppe -(CH₂)ₙOH darstellen, wobei n die Zahlen ab etwa 2 oder etwa 3 darstellt, mit der Maßgabe, dass mindestens einer der Reste R' den oben genannten Rest -(CH₂)ₙOH darstellt,
   ∘ insbesondere jene Verbindungen, die unter den INCI-Bezeichnungen Cocamid-MEA und/oder Cocamid-MIPA bekannt sind;
- C8-C30-Fettsäuremono- und -diester von Anlagerungsprodukten von etwa 1 bis etwa 30 Mol Ethylenoxid mit Glycerin, insbesondere die unter der INCI-Bezeichnung PEG-7-Glycerylcocoat bekannte Verbindung; und/oder Anlagerungsprodukte von etwa 4 bis etwa 30 Mol Ethylenoxid und/oder etwa 0 bis etwa 5 Mol Propylenoxid mit linearen Fettalkoholen, die etwa 8 bis etwa 22 C-Atome enthalten.
- Fettsäureester von Zuckern und Anlagerungsprodukte von Ethylenoxid mit Fettsäureestern von Zucker,
- Anlagerungsprodukte von Ethylenoxid mit Fettsäurealkanolamiden und Fettaminen,
- Alkyl(oligo)glucoside, beispielsweise Verbindungen mit der allgemeinen Formel RO-[G]x, wobei [G] vorzugsweise von Aldosen und/oder Ketosen abgeleitet ist, die etwa 5 bis etwa 6 Kohlenstoffatome enthalten, vorzugsweise von Glucose.
   ∘ Der Index x stellt den Oligomerisierungsgrad (DO) dar, d.h. die Verteilung der Mono- und Oligoglycoside. Der Index x hat vorzugsweise einen Wert im Bereich von etwa 1 bis etwa 10, beispielsweise im Bereich von etwa 1 bis etwa 3, wobei er diesbezüglich keine ganze Zahl sein muss, sondern eine Bruchzahl sein kann, die analytisch bestimmt werden kann.
   ∘ Geeignete Alkyl(oligo)glycoside haben einen Oligomerisierungsgrad zwischen etwa 1,2 und etwa 1,5.
   ∘ Der Rest R stellt geeigneterweise mindestens einen Alkyl- und/oder Alkenylrest dar, der etwa 4 bis etwa 24 C-Atome enthält.
   ∘ Beispiele für Alkyl(oligo)glucoside sind unter den INCI-Bezeichnungen Caprylyl/Caprylglucosid, Decylglucosid, Laurylglucosid und Cocoglucosid bekannt,
- Gemische von Alkyl(oligo)glucosiden und Fettalkoholen,
- Anlagerungsprodukte von etwa 5 bis etwa 60 Mol Ethylenoxid mit Rizinusöl oder hydriertem Rizinusöl, beispielsweise hydriertes PEG-40-Rizinusöl,
- Teilester von Polyolen mit etwa 3 bis etwa 6 Kohlenstoffatomen mit gesättigten Fettsäuren mit etwa 8 bis etwa 22 C-Atomen,
- Sterine:Unter "Sterinen" ist eine Gruppe von Steroiden zu verstehen, die eine Hydroxygruppe an C3 des Steroidrückgrats tragen und sowohl aus tierischem Gewebe (Zoosterole) als auch aus pflanzlichen Fetten (Phytosterole) isoliert sind. Beispiele für Zoosterine sind Cholesterin und Lanosterin. Beispiele für geeignete Phytosterine sind Ergosterin, Stigmasterin und Sitosterin. Sterine werden auch aus Pilzen und Hefen isoliert; diese sind als Mykosterine bekannt.
- Phospholipide:Dies sind in erster Linie Glucosephospolipide, die beispielsweise als Lecithine oder Phosphatidylcholine aus Eigelb oder aus Pflanzensamen (z.B. Sojabohnen) gewonnen werden.
- Hydriertes Rizinusöl.

Bevorzugte Fettalkoholethoxylate sind solche, bei denen der Fettalkoholanteil des Fettalkoholethoxylats eine Alkylkettenlänge von C4-C30, vorzugsweise C6-C25, bevorzugt C8-C20 aufweist, und/oder bei denen die Anzahl der Ethoxygruppen im Fettalkoholethoxylat 2 bis 120, vorzugsweise 4 bis 100, stärker bevorzugt 6 bis 80, stärker bevorzugt 8 bis 60, am stärksten bevorzugt 10 bis 40, beträgt.

Vorzugsweise ist das oder jedes Fettalkoholethoxylat ausgewählt aus Ethoxylaten von Beheneth, Ceteareth, Ceteth, Pareth und Deceth, vorzugsweise von Ceteareth, stärker bevorzugt von Ceteareth-20. Vorzugsweise ist das Fettalkoholpropoxylat PPG-3-Caprylylether.

Ein anderes geeignetes Alkoxylat ist eines, das ein Alkoxylat eines Nicht-Fettalkohols ist. Beispielsweise ist PPG-3-Benzylethermyristat ein alkoxyliertes Derivat von Benzylalkohol und als nichtionisches Tensid nützlich.

Eine Mischung der folgenden Tenside in einer Conditionerzusammensetzung ist besonders vorteilhaft: Quaternium-87, Behenoyl-PG-Trimoniumchlorid, Distearoylethylhydroxyethylmoniummethosulfat- oder chlorid und Behentrimoniumchlorid.

Wie dem Fachmann wohl bekannt sein wird, ist es üblich, eine oder mehrere Fettverbindungen in kosmetische Zusammensetzungen einzubauen. Unter Fettverbindungen im Sinne der vorliegenden Erfindung sind vorzugsweise aus natürlichen Quellen erhältliche Fettsäuren, Fettalkohole, Wachse, die sowohl in fester Form als auch als Flüssigkeiten in wässriger Dispersion vorliegen können, sowie Ölkomponenten zu verstehen.

Die hierin offenbarten Haarbehandlungsmittel können als optionale Komponente mindestens einen gesättigten oder ungesättigten, verzweigten oder unverzweigten C8-C30-Alkohol umfassen.

Beispiele für geeignete Fettalkohole sind unter anderem C12-13-Alkohole, C12-15-Alkohole, C12-16-Alkohole, C14-22-Alkohole, C16-17-Isoalkohole, C20-22-Alkohole, Arachidylalkohol, Cetylalkohol, Cetearylalkohol, Myristylalkohol, Behenylalkohol, Hexyldecycloctadecanol, Tetradecyleicosanol, Kokosnussalkohol, Isocetylalkohol, Isostearylalkohol, Stearylalkohol und Laurylalkohol. Vorzugsweise kann der Fettalkohol aus der Gruppe ausgewählt sein, bestehend aus Arachidylalkohol, Cetylalkohol, Cetearylalkohol, Myristylalkohol, Behenylalkohol, Hexyldecycloctadecanol, Tetradecyleicosanol, Kokosnussalkohol, Isocetylalkohol, Isostearylalkohol, Stearylalkohol und Laurylalkohol. Stärker bevorzugt kann der Fettalkohol Cetylalkohol, Stearylalkohol oder Gemische davon sein.

Die hierin offenbarten Haarbehandlungsmittel können ferner als optionale Komponente gesättigte oder ungesättigte, verzweigte oder unverzweigte C8-C30-Carbonsäuren und/oder deren Salze, vorzugsweise C10-C22-Carbonsäuren und/oder deren Salze und insbesondere Kokosnusssäuren, Laurinsäure, Myristinsäure, Palmitinsäure, Stearinsäure, Beheninsäure, Ölsäure und Gemische davon und/oder die Salze dieser Säuren umfassen. Besonders geeignet sind Stearinsäure und Palmitinsäure und/oder die Salze dieser Säuren, insbesondere Stearinsäure und Palmitinsäure.

Die hierin offenbarten Haarbehandlungsmittel können als optionale Komponente umfassen:
- mindestens einen Mono-, Di- oder Triester von Glycerin und eine Fettsäure mit 8 bis 30 Kohlenstoffatomen, und/oder
- mindestens einen Mono- oder Diester von Ethylenglykol und eine Fettsäure mit 8 bis 30 Kohlenstoffatomen.
Die C8-C30-Komponente der Fettsäure kann gesättigt oder ungesättigt, verzweigt oder unverzweigt sein. Geeignete Beispiele sind unter anderem Glycerylstearat und Glycoldistearat.

Die Gesamtmenge an Fettverbindungen, die gegebenenfalls in den hierin offenbarten Zusammensetzungen enthalten ist, liegt im Bereich von 0,1 bis 35 Gew.-%, bevorzugt 0,2 bis 30 Gew.-%, bevorzugter von 0,5 bis 20 Gew.-%, weiter bevorzugt von 1 bis 15 Gew.-%; am meisten bevorzugt 3 bis 12 Gew.-%, jeweils bezogen auf das Gewicht der Zusammensetzung.

Alternativ oder zusätzlich zu den oben beschriebenen Fettverbindungen kann das Haarbehandlungsmittel der vorliegenden Erfindung auch mindestens ein Fett oder Öl mit einem Schmelzpunkt von 20 °C oder höher umfassen. Im Zusammenhang mit einem festen Fett oder Öl bedeutet "fest", dass die Verbindung einen Schmelzpunkt von 20 °C oder höher hat. Vorzugsweise hat das oder jedes Fett oder Öl einen Schmelzpunkt von 25 °C oder höher oder 30 °C oder höher. Vorzugsweise ist Komponente (a) ein natürlich vorkommendes festes Fett oder Öl oder eine Mischung davon. Dies sind pflegende Substanzen, die dazu beitragen, die Haut- und Haarstruktur gesund zu halten. Natürlich vorkommende Rohstoffe haben den Vorteil, dass sie nachwachsen und somit nachhaltig genutzt werden können. Dieser Aspekt wird auch für viele Benutzer immer wichtiger.

Feste Fette und Öle sind unter anderem Fettsäuren (Triglyceride); sowohl natürlich abgeleitete Produkte als auch hydrierte Derivate davon. Ebenfalls enthalten sind mit Glycerin veresterte Fettsäuren, die Mono-, Di- und Triglyceride ergeben.

Bevorzugte feste Fette und Öle sind unter anderem ein festes pflanzliches Öl und/oder feste pflanzliche Butter. Natürlich vorkommende Rohstoffe haben, wie bereits erwähnt, den Vorteil, dass sie nachwachsen und somit nachhaltig genutzt werden können. Dieser Aspekt wird auch für viele Benutzer immer wichtiger. Darüber hinaus sind einige pflanzliche Öle oder Buttern, insbesondere wenn sie bei niedrigen Temperaturen schonend erhalten werden, äußerst wirksame Haut- und Haarpflegeprodukte, da sie auch eine Vielzahl bestimmter sekundärer Inhaltsstoffe wie Vitamine enthalten. Besonders bevorzugt sind pflanzliche Buttern mit einem Schmelzpunkt im Bereich von 20 °C bis 35 °C, wie Sheabutter (INCI-Bezeichnung: Butyrospermum-Parkii-(Shea-)Butter), Mangobutter (INCI-Bezeichnung: Butyrospermum-Parkii-(Shea-)Butter), Mangifera-Indica-(Mango- )Samenbutter), Murumuru-Butter (INCI-Bezeichnung: Astrocaryum-Murumuru-Samenbutter), Kakaobutter (INCI-Bezeichnung: Theobroma-Cacao-(Kakao-)Samenbutter) und/oder Cupuacu-Butter (INCI-Bezeichnung: Theobroma-Grandiflorum-Samenbutter), Cocos-Nucifera-(Kokosnuss- )Öl, Elaeis-Guineensis-(Palm-)Kernöl, Japan-Wachs, synthetisches Japan-Wachs, Ricinus-Communis-(Rizinus-)Samenöl, Simmondsia-Chinensis-(Jojoba-)Butter und Gemische davon. Besonders bevorzugt ist Sheabutter (INCI-Bezeichnung: Butyrospermum-Parkii-(Shea-)Butter).

Wie dem Fachmann wohl bekannt sein wird, werden Haarbehandlungsmittel, insbesondere Shampoos, Conditioner und sogenannte Treatments, häufig als Öl-in-Wasser-Emulsionen, wässrige, wässrig-alkoholische oder wässrig-glykolische Lösungen formuliert. Die Haarbehandlungsmittel der vorliegenden Erfindung können daher in Form von Öl-in-Wasser-Emulsionen, wässrigen, wässrig-alkoholischen, wässrig-glykolischen Lösungen oder in Form von Essenzen vorliegen. Der Wassergehalt solcher Zusammensetzungen kann mindestens 50 Gew.-%, bezogen auf das Gesamtgewicht der Zusammensetzung, betragen, geeignet mindestens 55 Gew.- %. Ein geeigneter Bereich für den Wassergehalt liegt zwischen 60 und 99 Gew.-%, bevorzugt 70 und 97 Gew.-%, bevorzugter 80 bis 96 Gew.-% und am meisten bevorzugt 85 bis 95 Gew.-%, bezogen auf das Gesamtgewicht des Haarbehandlungsmittels.

Geeignete Verfahren zur Herstellung der erfindungsgemäßen Zusammensetzungen, beispielsweise in Form von Öl-in-Wasser-Emulsionen, sind dem Fachmann wohl bekannt.

Es hat sich erfindungsgemäß als besonders vorteilhaft erwiesen, wenn die hierin definierten Haarbehandlungsmittel in Form von Emulsionen, besonders bevorzugt als O/W-Emulsionen, oder in Form von Essenzen vorliegen.

In einer bevorzugten Ausführungsform sind erfindungsgemäße Haarbehandlungsmittel dadurch gekennzeichnet, dass sie in Form einer O/W-Emulsion mit einem mittleren Teilchendurchmesser D₅₀ von 10 bis 1000 nm vorliegen. Der mittlere Teilchendurchmesser der in der Emulsion vorliegenden Teilchen bzw. Tröpfchen kann beispielsweise mittels Laserbeugung bestimmt werden (Roland I. et. al.; Systematic characterization of oil-in-water emulsions for formulation design"; Int. J. of Pharmaceutics, 2003, 263, Seiten 85 bis 94). Unter transparenten O/W-Emulsionen werden Emulsionen verstanden, welche einen NTU-Wert (Nephelometrischer Trübungswert) von 0 bis 30, insbesondere von 0 bis 20, aufweisen. Der NTU-Wert steht hierbei als Messwert für die Transparenz und ist eine mit einem kalibrierten Nephelometer gemessenen Trübung der Emulsion. Getrübte Emulsionen weisen NTU-Werte von größer 30 auf, wogegen transparente Emulsionen NTU-Werte von 30 und kleiner besitzen. Die Bestimmung des NTU-Wertes der erfindungsgemäßen O/W-Emulsionen kann beispielsweise mittels eines Turbidimeters, wie in der Offenlegungsschrift WO2016012327 A2 beschrieben, erfolgen. O/W-Emulsionen, welche die zuvor angeführten Teilchengrößen aufweisen, werden im Rahmen der vorliegenden Erfindung auch als Mikroemulsionen bezeichnet.

Zusätzlich zu den oben beschriebenen Bestandteilen können die erfindungsgemäßen Haarzusammensetzungen mindestens einen Wirkstoff enthalten, der vorteilhafterweise aus der Gruppe ausgewählt ist, die Trimethylglycin (Betaine), kationische Polymere, Pflanzenextrakte mit dem Echinacea-Extrakt als Ausnahme, da dieser zu dem Aktivstoff zählt, Bitterstoffe, Polyole, Parfums, UV-Filter, Strukturierungsmittel wie Maleinsäure, Farbstoffe zum Färben der Zusammensetzung, Wirkstoffe wie Bisabolol und/oder Allantoin, Antioxidantien, Konservierungsmittel wie Sorbitan Caprylate, Natriumbenzoat oder Salicylsäure, zusätzliche Viskositätsregler wie Salze (NaCl) oder Polymere und pH-Regler umfasst.

Trimethylglycin wirkt und irritationsmildernd und es ermöglicht insbesondere bei erfindungsgemäßen Haarbehandlungsmitteln, die als Shampoo vorliegen, die Reduktion von Tensidmengen bei gleichbleibend guten Schaummengen und Schaumeigenschaften. Zudem dient es aufgrund seiner Feuchtigkeitsspendenden Eigenschaften als zusätzliche haarkonditionierende Komponente - insbesondere als ein die Haarfasern verstärkender Wirkstoff.

Trimethylglycin kann in den erfindungsgemäßen Haarbehandlungsmitteln vorzugsweise in einem Gewichtsanteil von 0,01 bis 5 Gew.-%, mehr bevorzugt 0,05 bis 3 Gew.-% und insbesondere 0,1 bis 1,5 Gew.-% am Gesamtgewicht der Haarbehandlungsmittel eingesetzt werden.

Falls in den erfindungsgemäßen Haarbehandlungsmitteln vorhanden, sind unter geeigneten kationischen Polymeren vorzugsweise kationische Polymere natürlichen Ursprungs zu verstehen, wobei diese kationischen Polymere nicht zu den Verdickern zählen. Bevorzugte kationische Polymere im Sinne der vorliegenden Erfindung sind:
- quaternisierte Cellulose-Derivate, wie sie unter den Bezeichnungen Celquat^{®} und Polymer JR^{®} im Handel erhältlich sind,
- kationische Stärke-Derivate, wie sie beispielsweise unter der Bezeichnung Mirustyle^{®} im Handel erhältlich sind,
- hydrophob modifizierte Cellulosederivate, beispielsweise die unter dem Handelsnamen SoftCat^{®} vertriebenen kationischen Polymere,
- kationische Alkylpolyglycoside,
- kationisierter Honig, beispielsweise das Handelsprodukt Honeyquat^{®} 50,
- kationische Guar-Derivate, wie insbesondere die unter den Handelsnamen Cosmedia^{®}Guar N-Hance^{®} und Jaguar^{®} vertriebenen Produkte
- kationische Cassia - und/oder Inulin-Derivate wie die unter den INCI-Bezeichnungen Cassia Hydroxypropyltrimonium Chloride und Hydroxypropyltrimonium Inulin bekannten kationischen Polymere.

Besonders bevorzugt sind die unter den INCI-Bezeichnungen Guar Hydroxypropyltrimonium Chloride, Polyquaternium-10, Polyquaternium-67, Hydroxypropyltrimonium Inulin und/oder Hydroxypropyltrimonium Hydrolyzed Corn Starch bekannten kationischen Polymere; insbesondere bevorzugt sind Guar Hydroxypropyltrimonium Chloride und Polyquaternium-10.

Die erfindungsgemäßen Haarbehandlungsmittel können vorzugsweise mindestens ein kationisches Polymer in einem Gewichtsanteil von 0,01 bis 1 Gew.-%, mehr bevorzugt 0,02 bis 0,75 Gew.-% und insbesondere 0,03 bis 0,5 Gew.-% am Gesamtgewicht der Haarbehandlungsmittel enthalten.

Unter geeigneten Polyolen (d.h. physiologisch verträglichen Polyalkoholen oder Polyhydroxylverbindungen) werden vorzugsweise Polyole mit 2 bis 20 Kohlenstoffatomen und mindestens zwei Hydroxylgruppen verstanden.

Typische Beispiele sind geradkettige, verzweigte oder cyclische Alkanole mit 2 bis 10, vorzugsweise mit 2 bis 6 Kohlenstoffatomen, wie 1,2-Propandiol 1,3-Butylenglycol, Dipropylenglycol, Glycerin und/oder Diglycerin, Glykole wie Polyethylenglykole und/oder Polypropylenglykole sowie Zuckeralkohole (Alditole), Zucker oder Zuckerderivate wie Mannit, Isomaltit, Lactit, Sorbit, Xylit, Fructose, Glucose, Lactose, Galactose und/oder Saccharose.

Besonders bevorzugt sind C₂-C₆-Polyole, vorzugsweise Alditole wie Mannit, Isomaltit, Lactit, Sorbit und/oder Xylit, 1,2-Propylenglycol, 1,3-Butylenglycol, Dipropylenglycol, Glycerin und/oder Diglycerin, besonders bevorzugt 1,2-Propylenglycol, 1,3-Butylenglycol, Dipropylenglycol, Glycerin und/oder Diglycerin, insbesondere bevorzugt ist Glycerin.

Die erfindungsgemäßen Haarbehandlungsmittel enthalten Polyole - bevorzugt Glycerin - vorzugsweise in einem Gewichtsanteil von 0,01 bis 10 Gew.-%, mehr bevorzugt 0,05 bis 7,5 Gew.- % und insbesondere 0,1 bis 5 Gew.-% am Gesamtgewicht der Haarbehandlungsmittel.

Unter geeigneten Pflanzenextrakten sind Extrakte zu verstehen, die aus allen Teilen einer Pflanze hergestellt werden können. Normalerweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Die am besten geeigneten Extrakte sind Paeonia lactiflora, Rosa-damascena-Blume, Malus-domestica-Frucht, Argania-spinosa-Schalenpulver, Laminaria saccharina, Cannabis sativa, Grüntee, Eichenrinde, Brennnessel, Hamamelis, Hopfen, Kamille, Klettenwurzel, Schachtelhalm, Weißdorn, Lindenblume, Litschi, Mandel, Aloe vera, Fichtennadel, Rosskastanie, Sandelholz, Wacholder, Kokosnuss, Mango, Aprikose, Limette, Weizen, Kiwi, Melone, Orange, Grapefruit, Salbei, Rosmarin, Birke, Malve, Kuckucksblume, Quendel, Schafgarbe, Thymian, Zitronenmelisse, Hemlocktanne, Huflattich, Eibisch, Ginseng, Ingwerwurzel, Olea europea, Boerhavia-diffusa-Wurzeln, Foeniculum vulgaris und Apim graveolens. Die Extrakte aus Paeonia lactiflora, Rosa-damascena-Blume, Malus-domestica-Frucht, Argania-spinosa-Schalenpulver, Laminaria saccharina, Cannabis sativa, Grüntee, Brennnessel, Hamamelis, Kamille, Aloe vera, Ginseng, Olea europea und/oder Boerhavia diffusa sind für die Verwendung in den erfindungsgemäßen Zusammensetzungen besonders bevorzugt. Wasser, Alkohole und ihre Gemische können als Extraktionsmittel zur Herstellung der erwähnten Pflanzenextrakte verwendet werden. Unter den Alkoholen sind dabei niedere Alkohole wie Ethanol und Isopropanol besonders bevorzugt, insbesondere aber mehrwertige Alkohole wie Ethylenglykol und Propylenglykol, sowohl als alleiniges Extraktionsmittel als auch in Mischung mit Wasser. Pflanzenextrakte auf Basis von Wasser/Propylenglykol im Verhältnis 1:10 bis 10:1 haben sich als besonders geeignet erwiesen. Die Pflanzenextrakte können sowohl in reiner als auch in verdünnter Form eingesetzt werden. Sofern sie in verdünnter Form eingesetzt werden, enthalten sie üblicherweise ca. 2-80 Gew.-% Aktivwirkstoff und als Lösungsmittel das bei ihrer Gewinnung eingesetzte Extraktionsmittel oder Extraktionsmittelgemisch. Die Pflanzenextrakte können in den erfindungsgemäßen Haarbehandlungsmitteln (bezogen auf das Gesamtgewicht der Produkte) bevorzugt in einer Menge von 0,01 bis 10 Gew.-%, stärker bevorzugt 0,05 bis 7,5 Gew.-% und insbesondere 0,1 bis 5 Gew.-% eingesetzt werden.

Das hierin offenbarte Haarbehandlungsmittel kann ebenso ein Parfüm enthalten. Vorzugsweise ist ein Parfüm in einer Menge in einem Bereich von 0,1 bis 0,8 Gew.-%, bevorzugt 0,2 bis 0,6 %, bezogen auf das Gewicht des Haarbehandlungsmittels, enthalten.

Ein Bitterstoff ist in Kosmetika, Haushaltsprodukten usw., die so hergestellt sind, dass ihre Form, Farbe, Haptik usw. kleine Kinder oder Babys ansprechen und zum Spielen anregen, was auch zum Verschlucken führen kann, besonders wichtig. Ein Bitterstoff verhindert dies. Denatoniumbenzoat ist ein extrem starker Bitterstoff und daher auch bei sehr geringen Konzentrationen besonders wirksam. Darüber hinaus sind damit keine bekannten unerwünschten Wirkungen assoziiert.

Unter geeigneten pH-Reglern, die zur Verwendung in den erfindungsgemäßen Zusammensetzungen besonders bevorzugt sind, sind Zitronensäure, Milchsäure, Äpfelsäure, Glykolsäure, insbesondere Zitronensäure und/oder Milchsäure. Der pH-Regler kann auch eine Base wie Alkanolamine und/oder Natriumhydroxid sein.

Durch Verwendung des erfindungsgemäßen Haarbehandlungsmittels ist es möglich, auf organische Siliziumverbindungen, insbesondere Silikone, zu verzichten. In dem Zusammenhang der vorliegenden Erfindung bedeutet "silikonfrei", dass die Haarbehandlungsmittel frei von organische Siliziumverbindungen, insbesondere frei von Silikonen, Trisiloxanen und Silikonölen, ist.

Die Erfindung betrifft auch ein Shampoo, das das hierin offenbarte Haarbehandlungsmittel umfasst.

Die Erfindung betrifft auch einen Conditioner, der das hierin offenbarte Haarbehandlungsmittel umfasst. Der Conditioner eignet sich zur Pflege von menschlichem Haar, insbesondere zur Verwendung nach der Haarreinigung als Zusammensetzung ohne Ausspülen oder zum Ausspülen. Das hierin offenbarte Haarbehandlungsmittel kann auch bei Haarbehandlungen und Haarpackungen nützlich sein.

Die Ausdrücke "ohne Ausspülen" und "zum Ausspülen" bedeuten, dass das Haarbehandlungsmittel entweder für eine relativ kurze Zeit im Haar verbleibt, beispielsweise für weniger als eine Minute, falls erforderlich, oder für einige Minuten oder eine Stunde, bis sie wieder ausgespült wird, oder dass die Zusammensetzung bis zur nächsten Haarwäsche im Haar bleibt, was einige Tage dauern kann. Beide haben gewisse Vorteile. Bleibt die Zusammensetzung längere Zeit auf dem Haar, kann das volle Pflegepotential aller Inhaltsstoffe ausgeschöpft werden, während eine in kurzer Zeit auszuspülende Zusammensetzung auch Inhaltsstoffe enthalten kann, die eine gute Pflegewirkung haben, deren längeres Verbleiben in den Haaren jedoch unangenehm wäre. Im Sinne der vorliegenden Erfindung sind Zusammensetzungen zum Ausspülen bevorzugt.

Die vorliegende Erfindung bezieht sich auch auf ein Verfahren zur Verbesserung der Haarpflege, insbesondere zur Verbesserung der antistatischen Eigenschaften, der Weichheit, der Kämmbarkeit und/oder Entwirrbarkeit von Haaren, umfassend das Aufbringen eines erfindungsgemäßen Haarbehandlungsmittels auf das Haar. Das Verfahren beinhaltet das Aufbringen des hierin offenbarten Haarbehandlungsmittels auf das Haar. Das Verfahren beinhaltet das Abgeben des hierin offenbarten Haarbehandlungsmittels auf die Hand des Benutzers und von der Hand des Benutzers auf das Haar. Das Verfahren kann zusätzlich ein Reiben des Haars beinhalten. Das Verfahren kann beinhalten, dass der Benutzer das Haarbehandlungsmittel vor der Aufbringung auf das Haar in seiner Hand oder seinen Händen verteilt.

Das hierin offenbarte Verfahren kann ebenso zusätzliche, optionale Schritte beinhalten. In einem Beispiel können derartige Schritte ein Lufttrocknen des Haares, ein Trocknen des Haares unter Verwendung von Wärme, Frisieren des Haares und einen beliebigen anderen geeigneten Schritt beinhalten, der einem Fachmann auf dem Gebiet der Haarbehandlung bekannt ist.

Die Erfindung kann auch durch die nachfolgenden Aussagen definiert werden:
1. Ein Haarbehandlungsmittel enthaltend
   a) mindestens ein Reaktionsprodukt, erhältlich durch Umsetzung von
      i) Polyglycerin oder einem Polyglycerinester,
      ii) Trimethylglycin und
      iii) mindestens einer Säure, und
   b) mindestens einen Aktivstoff.
2. Ein Haarbehandlungsmittel nach Aussage 1, wobei das Polyglycerin i) 1,1 bis 30 Glycerineinheiten, vorzugsweise 1,1 bis 10, mehr bevorzugt 1,1 bis 6, besonders bevorzugt 2 bis 4 und insbesondere 3 Glycerineinheiten aufweist.
3. Ein Haarbehandlungsmittel nach einer vorhergehenden Aussage, wobei das Polyglycerin i) aus unter den INCI-Bezeichnungen Polyglycerine-2, Polyglycerine-3, Polyglycerine-4, Polyglycerine-5, Polyglycerine-6, Polyglycerine-7, Polyglycerine-8, Polyglycerine-9, Polyglycerine-10 bekannten Verbindungen, vorzugsweise aus Polyglycerine-2, Polyglycerine-3, Polyglycerine-4, Polyglycerine-5 und/oder Polyglycerine-6 und insbesondere aus Polyglycerine-3 ausgewählt ist.
4. Ein Haarbehandlungsmittel nach einer vorhergehenden Aussage, wobei der Polyglycerinester i) gebildet wird aus Polyglycerin mit 1,1 bis 30 Glycerineinheiten und mindestens einer geradkettigen oder verzweigten, gesättigten oder ungesättigten C₆-C₃₀-Carbonsäure.
5. Ein Haarbehandlungsmittel nach einer vorhergehenden Aussage, wobei der Polyglycerinester i) ein Monoester, Diester, Triester, Tertraester usw. sein und aus 1,1 bis 30 Glycerineinheiten umfassendem Polyglycerin mit Behensäure, Caprinsäure, Caprylsäure, Olivensäuren, Kokossäuren, Linolsäure, Dilinolsäure, Myristinsäure, Stearinsäure, Isostearinsäure, Hydroxystearinsäure, Laurinsäure, Ölsäure, Palmitinsäure, Erucasäure, Palmsäuren, Ricinolsäure, Decansäure, Undecylensäure oder Mischungen davon ist.
6. Ein Haarbehandlungsmittel nach einer vorhergehende Aussage, wobei der Polyglycerinester i) ausgewählt ist aus unter den INCI-Bezeichnungen Polyglyceryl-2 Caprate, Polyglyceryl-2 Dicaprate, Polyglyceryl-3 Caprate, Polyglyceryl-3 Tricaprate, Polyglyceryl-4 Caprate, Polyglyceryl-5 Caprate, Polyglyceryl-6 Caprate, Polyglyceryl-10 Caprate, Polyglyceryl-2 Caprylate, Polyglyceryl-2 Dicaprylate, Polyglyceryl-3 Caprylate, Polyglyceryl-3 Tricaprylate, Polyglyceryl-4 Caprylate, Polyglyceryl-5 Caprylate, Polyglyceryl-6 Caprylate, Polyglyceryl-10 Caprylate, Polyglyceryl-2 Cocoate, Polyglyceryl-2 Dicocoate, Polyglyceryl-3 Cocoate, Polyglyceryl-3 Triccocoate, Polyglyceryl-4 Cocoate, Polyglyceryl-5 Cocoate, Polyglyceryl-6 Cocoate, Polyglyceryl-10 Cocoate, Polyglyceryl-2 Stearate, Polyglyceryl-2 Distearate, Polyglyceryl-3 Stearate, Polyglyceryl-3 Tristearate, Polyglyceryl-4 Stearate, Polyglyceryl-5 Stearate, Polyglyceryl-6 Stearate, Polyglyceryl-10 Stearate, Polyglyceryl-2 Isostearate, Polyglyceryl-2 Diisostearate, Polyglyceryl-3 Isostearate, Polyglyceryl-3 Triisostearate, Polyglyceryl-4 Isostearate, Polyglyceryl-5 Isostearate, Polyglyceryl-6 Isostearate, Polyglyceryl-10 Isostearate, Polyglyceryl-2 Ricinoleate, Polyglyceryl-2 Diricinoleate, Polyglyceryl-3 Ricinoleate, Polyglyceryl-3 Triricinoleate, Polyglyceryl-3 Polyricinoleate, Polyglyceryl-4 Ricinoleate, Polyglyceryl-5 Ricinoleate, Polyglyceryl-6 Ricinoleate, Polyglyceryl-10 Ricinoleate, Polyglyceryl-2 Oleate, Polyglyceryl-2 Dioleate, Polyglyceryl-3 Oleate, Polyglyceryl-3 Trioleate, Polyglyceryl-4 Oleate, Polyglyceryl-5 Oleate, Polyglyceryl-6 Oleate, Polyglyceryl-10 Oleate bekannten Verbindungen.
7. Ein Haarbehandlungsmittel nach Aussage 6, wobei a)i) Polyglycerine-3 und/oder Polyglyceryl-3 Oleate umfasst.
8. Ein Haarbehandlungsmittel nach einer vorhergehenden Aussage, wobei die mindestens eine Säure a)iii) ausgewählt ist aus organischen Säuren aus der Gruppe der geradkettigen oder verzweigten, gesättigten oder ungesättigten C₁-C₂₄-Mono-, Di- oder Tricarbonsäuren, welche eine oder mehrere Hydroxylgruppen als Substituenten tragen können und/oder der aromatischen Säuren.
9. Ein Haarbehandlungsmittel nach einer vorhergehenden Aussage, wobei die Säure a)iii) ausgewählt ist aus Ameisensäure, Essigsäure, Propionsäure, Caprylsäure, Caprinsäure, Benzoesäure, Zitronensäure, Oxalsäure, Weinsäure, (Poly)acrylsäure, Fumarsäure, Maleinsäure, Itaconsäure, Glykolsäure, Gluconsäure, Äpfelsäure, Milchsäure, Mandelsäure, Tiglinsäure, Succinsäure oder Ascorbinsäure.
10. Ein Haarbehandlungsmittel nach einer vorhergehenden Aussage, wobei die Säure a)iii) Essigsäure ist.
11. Ein Haarbehandlungsmittel nach einer vorhergehenden Aussage, wobei das Reaktionsprodukt
   a) erhältlich ist durch Reaktion von
      i) Polyglycerine-3 und/oder Polyglyceryl-3 Oleate,
      ii) Trimethylglycin und
      iii) Essigsäure.
12. Ein Haarbehandlungsmittel enthaltend
   A) mindestens eine Verbindung der allgemeinen Formel (I)

      R¹(OCH₂CH(OR²)CH₂)ₓ-O-R³ (I)

      in der
      - R¹, R², R³ unabhängig voneinander für -H, -C(O)-CH₂-N⁺(CH₃)₃ oder für einen geradkettigen oder verzweigten, gesättigten oder ungesättigten, -OH oder NH₂-Substituenten tragenden C₆-C₃₀-Acylrest stehen,
      - x für eine Zahl von 1,1 bis 30 steht,
      - wobei mindestens einer der Reste R¹, R², R³ für die Gruppierung -C(O)CH₂-N⁺(CH₃)₃ steht, und
      - wobei Formel (I), eine der Anzahl der darin enthaltenen positiven Ladungen entsprechende Anzahl an physiologisch akzeptablen Anionen A⁻ enthält, und
   B) mindestens einen Aktivstoff.
13. Ein Haarbehandlungsmittel nach einer der Aussagen 1 oder 12, enthaltend Verbindungen der Formel (I), in der
   - einer der Reste R¹, R³ für -C(O)-CH₂-N⁺(CH₃)₃ und der andere für -H, -C(O)-CH₂-N⁺(CH₃)₃ oder für einen geradkettigen oder verzweigten, gesättigten oder ungesättigten, -OH oder NH₂-Substituenten tragenden C₆-C₂₀-Acylrest,
   - R² für -H, und
   - x für eine Zahl von 1,1 bis 10 steht.
14. Ein Haarbehandlungsmittel nach einer der Aussagen 1, 12 oder 13, enthaltend Verbindungen der Formel (I), in der
   - einer der Reste R¹, R³ für -C(O)-CH₂-N⁺(CH₃)₃ und der andere für -H, -C(O)-CH₂-N⁺(CH₃)₃ oder für Heptanoat, Caprylat, Nonanoat, Caprat, Undecylat, Laurat, Tridecanoat, Myristat, Pentadecanoat, Palmitat, Heptadecanoat, Stearat, Isostearat, Oleat, Ricinoleat, Nonadecanoat, Arachidat, Behenat,
   - R² für -H, und
   - x für eine Zahl von 1,1 bis 6, besonders bevorzugt von 2 bis 4 und insbesondere für 3 steht.
15. Ein Haarbehandlungsmittel nach einer der Aussagen 1 oder 12 bis 14, enthaltend mindestens einen Betainester der allgemeinen Formel (la)

   [R¹(OCH₂CH(OR²)CH₂)ₓ-O-C(O)-CH₂-N⁺(CH₃)₃] A⁻ (la)

   in der
   - R¹ für -H, -C(O)-CH₂-N⁺(CH₃)₃ oder für Caprylat, Caprat, Laurat, Myristat, Palmitat, Stearat, Isostearat, Oleat, Ricinoleat, Arachidat, Behenat,
   - R² für -H,
   - x für eine Zahl von 1,1 bis 6, bevorzugt von 2 bis 4 und insbesondere für 3 und
   - A⁻ für ein physiologisch akzeptables organisches Anion wie Formiat, Acetat, Propionat, Benzoat, Citrat, Oxalat, Tartat, (Poly)acrylat, Fumarat, Maleat, Lactat, Itaconat, Glykolat, Gluconat, Malat, Mandelat, Tiglat, Succinat, Ascorbat steht.
16. Ein Haarbehandlungsmittel nach einer der Aussagen 1 oder 12 bis 15, enthaltend Betainester der Formel (la), in der
   - R¹ für -H, -C(O)-CH₂-N⁺(CH₃)₃ oder für Oleat,
   - R² für -H,
   - x für eine Zahl von 1,1 bis 6, besonders bevorzugt von 2 bis 4 und insbesondere für 3 und
   - A⁻ für Formiat, Acetat, Propionat, Benzoat, Citrat, Oxalat, Tartat, Lactat oder Malat steht.
17. Ein Haarbehandlungsmittel nach einer vorhergehenden Aussage, enthaltend als Komponente(n) a) oder A) eine unter der INCI-Bezeichnung Polyglyceryl-3 betainate acetate bekannte Verbindung.
18. Ein Haarbehandlungsmittel nach einer vorhergehenden Aussage, enthaltend die Komponente(n) a) oder A) in einer Menge von 0,01 bis 10,0 Gew.-%, mehr bevorzugt von 0,05 bis 5,0 Gew.-% und besonders bevorzugt von 0,1 bis 3,0 Gew.-% am Gesamtgewicht des Haarbehandlungsmittels.
19. Ein Haarbehandlungsmittel nach einer vorhergehenden Aussage, wobei der mindestens eine Aktivstoff b) ausgewählt ist aus der Gruppe bestehend aus einem Xanthin, einem Taurin, einem Vitamin, einem Antischuppenmittel, einem Echinacea-Pflanzenextrakt, einem Mikrobiom-Aktivmittel und einer Kombination davon.
20. Ein Haarbehandlungsmittel nach Aussage 19, wobei das Vitamin ein Vitamin, ein Provitamin oder eine Vitaminvorstufe ist, die vorzugsweise einer der Gruppen A, B, C, E, F und H zugeordnet werden kann.
21. Ein Haarbehandlungsmittel nach Aussage 19 oder Aussage 20, wobei das Vitamin ausgewählt ist aus der Gruppe bestehend aus 2,4-Dihydroxy-N-(3-hydroxypropyl)-3,3-dimethyl-butyramid, Provitamin B₅), Pantothensäure (Vitamin B₅), Niacin (Vitamin B₃), Nicotinamid (Vitamin B₃), L-Ascorbinsäure (Vitamin C), Thiamin (Vitamin B₁), Riboflavin (Vitamin B₂, Vitamin G), Biotin (Vitamin 87, Vitamin H), Folsäure (Vitamin B₉, Vitamin B₁₁ oder Vitamin M), Vitamin B₆ und Vitamin B12.
22. Ein Haarbehandlungsmittel nach Aussage 19, wobei das Taurin 2-Aminoethansulfonsäure oder ein Taurinderivat, ausgewählt aus der Gruppe bestehend aus N-Monomethyl-Taurin oder N,N-Dimethyl-Taurin ist.
23. Ein Haarbehandlungsmittel nach Aussage 19, wobei das Xanthin ausgewählt ist aus der Gruppe bestehend aus Coffein, Theophyylin, Theobromin und Mischungen davon, insbesondere Coffein, ist.
24. Ein Haarbehandlungsmittel nach Aussage 19, wobei das Antischuppenmittel ein anorganisches Zinksalz ist, vorzugsweise ein wasserunlösliches anorganisches Zinksalz, besonders bevorzugt Zinkpyrithion, Zinkaluminat, Zinkcarbonat, Zinkoxid, Calamine, Zinkphosphate, Zinkorthophosphat, Zinkpyrophosphat, Zinkselenid, Zinksulfid, Zinksilikat, Zinkorhtosilikat, Zinkmetalsilikat, Zinkborat, Zinkhydroxid und Zinkhydroxysulfat, und insbesondere Zinkoxid oder Zinkcarbonat ist.
25. Ein Haarbehandlungsmittel nach Aussage 19, wobei das Antischuppenmittel ausgewählt ist aus der Gruppe bestehend aus Piroctone Olamine, Climbazol, Ketoconazole, Salicylsäure, Schwefel, Selensulfid, Teerpräparate, Undecensäurederivate, Klettenwurzelextrakte, Pappelextrakte, Brennesselextrakte, Walnussschalenextrakte, Birkenextrakte, Weidenrindenextrakte, Rosmarinextrakte, Arnikaextrakte und Propandiol Caprylate, wobei das Antischuppenmittel insbesondere Propandiol Caprylate ist.
26. Ein Haarbehandlungsmittel nach Aussage 19, wobei das Mikrobiom-Aktivmittel einen Stoff darstellt, der aus einem Mikrobiom, insbesondere aus gramnegativen Bakterien, gewonnen wird.
27. Ein Haarbehandlungsmittel nach einer der Aussagen 19 oder 26, wobei das Mikrobiom-Aktivmittel Carnitin, ein Carnitin-Derivat oder ein Carnitinsalz ist, vorzugsweise L-Carnitin, Acetyl-L-Carnitin, L-Carnitin-Fumarat, L-Carnitin-Citrat, Lauroyl-L-Carnitin und L-Carnitin-Tartrat, insbesondere L-Carnitin-Tartrat.
28. Ein Haarbehandlungsmittel nach einer der Aussagen 19 bis 21, wobei das Vitamin, das Provitamin oder die Vitaminvorstufe in einer Menge von 0,001 bis 10 Gew.-%, bevorzugt von 0,01 bis 2 Gew.-% und bevorzugter von 0,1 bis 1 Gew.-% bezogen auf das Gesamtgewicht des Haarbehandlungsmittels in dem Haarbehandlungsmittel enthalten ist.
29. Ein Haarbehandlungsmittel nach einer der Aussagen 19, 24 oder 25, wobei das Antischuppenmittel in einer Menge von 0,01 bis 5 Gew.-%, bevorzugt 0,02 bis 4 Gew.-%, bevorzugter von 0,05 bis 3 Gew.-% und noch bevorzugter von 0,1 bis 1 Gew.-% bezogen auf das Gesamtgewicht des Haarbehandlungsmittels in dem Haarbehandlungsmittel enthalten ist.
30. Ein Haarbehandlungsmittel nach einer der Aussagen 19 26 oder 27, wobei das Mikrobiom-Aktivmittel in einer Menge von 0,001 bis 10 Gew.-%, bevorzugt von 0,01 bis 2 Gew.-% und bevorzugter von 0,1 bis 1 Gew.-% bezogen auf das Gesamtgewicht des Haarbehandlungsmittels in dem Haarbehandlungsmittel enthalten ist.
31. Ein Haarbehandlungsmittel nach einer der Aussagen 19 oder 22, wobei das Taurin oder das Taurin-Derivat in einer Menge von 0,001 bis 10 Gew.-%, bevorzugt von 0,01 bis 2 Gew.-% und bevorzugter von 0,1 bis 1 Gew.-% bezogen auf das Gesamtgewicht des Haarbehandlungsmittels in dem Haarbehandlungsmittel enthalten ist.
32. Ein Haarbehandlungsmittel nach einer der Aussagen 19 oder 23, wobei das Xanthin, insbesondere das Coffein, in einer Menge von 0,001 bis 10 Gew.-%, bevorzugt von 0,01 bis 2 Gew.-% und bevorzugter von 0,1 bis 1 Gew.-% bezogen auf das Gesamtgewicht des Haarbehandlungsmittels in dem Haarbehandlungsmittel enthalten ist.
33. Ein Haarbehandlungsmittel nach Aussage 19, wobei der Echinacea-Pflanzenextrakt in einer Menge von 0,001 bis 10 Gew.-%, bevorzugt von 0,01 bis 2 Gew.-% und bevorzugter von 0,1 bis 1 Gew.-% bezogen auf das Gesamtgewicht des Haarbehandlungsmittels in dem Haarbehandlungsmittel enthalten ist.
34. Ein Haarbehandlungsmittel nach einer vorhergehenden Aussage, enthaltend die Komponente(n) b) oder B) in einer Menge von 0,001 bis 5 Gew.-%, bevorzugt 0,005 bis 4 Gew.-%, bevorzugter 0,01 bis 3 Gew.-%, am meisten bevorzugt 0,05 bis 1,5 Gew.-% bezogen auf das Gesamtgewicht des Haarbehandlungsmittels.
35. Ein Haarbehandlungsmittel nach einer vorhergehenden Aussage, ferner umfassend 1,0 bis 50,0 Gew.-% mindestens eines anionischen, nichtionischen, amphoteren oder zwitterionischen Tensids.
36. Ein Haarbehandlungsmittel nach einer vorhergehenden Aussage, ferner umfassend mindestens ein anionisches Tensid und gegebenenfalls mindestens ein nichtionisches, amphoteres oder zwitterionisches Tensid.
37. Ein Haarbehandlungsmittel nach einer vorhergehenden Aussage, umfassend 1,0 bis 35,0 Gew.-% mindestens eines anionischen Tensids, bezogen auf das Gesamtgewicht des Haarbehandlungsmittels.
38. Ein Haarbehandlungsmittel nach einer vorhergehenden Aussage, umfassend 0,5 bis 15,0 Gew.-% mindestens eines zwitterionischen Tensids, bezogen auf das Gesamtgewicht des Haarbehandlungsmittels.
39. Ein Haarbehandlungsmittel nach einer vorhergehenden Aussage, umfassend ein zwitterionisches Tensid, umfassend Cocoamidopropylbetain.
40. Ein Haarbehandlungsmittel nach einer vorhergehenden Aussage, umfassend ein nichtionisches Tensid.
41. Ein Haarbehandlungsmittel nach einer vorhergehenden Aussage, umfassend 0,1 bis 15,0 Gew.- %, vorzugsweise 0,5 bis 10,0 Gew.-% und insbesondere 0,5 bis 5,0 Gew.-% mindestens eines kationischen Tensids, bezogen auf das Gesamtgewicht des Haarbehandlungsmittels.
42. Ein Haarbehandlungsmittel nach einer vorhergehenden Aussage, umfassend eines oder mehrere von: Quaternium-87, Behenoyl-PG-Trimoniumchlorid, Distearoylethylhydroxyethylmoniummethosulfat- oder chlorid und Behentrimoniumchlorid.
43. Ein Haarbehandlungsmittel nach einer vorhergehenden Aussage, die ein Haarshampoo ist.
44. Ein Haarbehandlungsmittel nach einer der Aussagen 1 bis 42, die ein Haarconditioner ist.
45. Ein Haarbehandlungsmittel nach Aussage 44, die ein Haarconditioner ohne Ausspülen ist.
46. Ein Haarbehandlungsmittel nach Aussage 44, die ein Haarconditioner zum Ausspülen ist.
47. Ein Haarbehandlungsmittel nach einer der Aussagen 1 bis 42, die ein Haarserum ist.
48. Ein Haarbehandlungsmittel nach einer vorhergehenden Aussage, ferner umfassend eine oder mehrere Fettverbindungen, vorzugsweise eine Fettsäure, einen Fettalkohol oder ein natürliches oder synthetisches Wachs ist.
49. Ein Haarbehandlungsmittel nach Aussage 48, wobei die Fettverbindung Sheabutter ist.
50. Ein Haarbehandlungsmittel nach einer vorhergehenden Aussage, ferner umfassend ein oder mehrere kationische Polymere.
51. Ein Haarbehandlungsmittel nach einer vorhergehenden Aussage, ferner umfassend mindestens einen Wirkstoff ausgewählt aus der Gruppe, umfassend Trimethylglycin (Betaine), kationische Polymere, Pflanzenextrakte außer der Echinacea-Pflanzenextrakt, Bitterstoffe, Polyole, Parfums, UV-Filter, Strukturierungsmittel wie Maleinsäure, Farbstoffe zum Färben der Zusammensetzung, Wirkstoffe wie Bisabolol und/oder Allantoin, Antioxidantien, Konservierungsmittel wie Sorbitan Caprylate, Natriumbenzoat oder Salicylsäure, zusätzliche Viskositätsregler wie Salze (NaCl) oder Polymere und pH-Regler.
52. Ein Verfahren zur Verbesserung der Haarpflege, umfassend das Aufbringen eines Haarbehandlungsmittels nach einer der Aussagen 1 bis 51 auf das Haar.
53. Verwendung eines Haarbehandlungsmittels nach einer der Aussagen 1 bis 51 zur Verbesserung der Haarpflege.

### Beispiele

Die folgenden Zusammensetzungen wurden hergestellt und es wurde überraschend festgestellt, dass sie dem Haar verbesserte antistatische Eigenschaften, mehr Weichheit sowie eine verbesserte Kämmbarkeit und/oder Entwirrbarkeit verleihen.

### Haarshampoo:

| | **1 [%]** | **2 [%]** | **3 [%]** | **4 [%]** |
|---|---|---|---|---|
| Polyglyceryl-3 betainate acetate* | 0,01 - 4 | 0,075 - 0,75 | 0,1 - 0,4 | 0,1 - 0,4 |
| Steardimonium Hydroxypropyl Hydrolyzed Keratin | 0,01 - 4 | 0,075 - 0,75 | 0,1 - 0,4 | 0,1 - 0,4 |
| anionisches Tensid | 0,5 - 12,5 | 0,5 - 12,5 | | |
| Sodium Laureth Sulfate | | | 1 - 10 | 1 - 10 |
| amphoteres Tensid | 0,1 - 5 | | | |
| Disodium Cocoamphodiacetate | | 0,1 - 5 | | 0,1 - 5 |
| Cocoamidopropylbetaine | | | 0,1 - 5 | 0,1 - 5 |
| nichtionisches Tensid | 0,1 - 5 | | | |
| Cocamide MEA | | | | 0,1 - 1 |
| Fett- oder Wachs | 0,01 - 5 | | | |
| Jojobaöl | | | | 0,01 - 1 |
| Aprikosenkernöl | | | 0,01 - 1 | |
| Carnithine | 0,01 - 2 | 0,01 - 2 | 0,01 - 2 | 0,1 - 0,6 |
| zwitterionisches und/oder amphoteres Tensid | 0,1 - 8 | 0,1 - 8 | | |
| Zitronensäure | 0,1 - 0,5 | 0,1 - 0,5 | 0,1 - 0,5 | 0,1 - 0,5 |
| Natriumchlorid | 0-1,5 | 0 - 1,5 | 0,1 - 1,5 | 0,1 - 1,3 |
| Wasser | ad 100 | ad 100 | ad 100 | Wasser |
| pH-Wert | 4,5 - 5,0 | 4,5 - 5,0 | 4,8 ± 0,2 | 4,8 ± 0,2 |

### Haarshampoo:

| **Shampoo** | **5 [%]** | **6 [%]** | **7 [%]** | **8 [%]** |
|---|---|---|---|---|
| Sodium Laureth Sulfate | 10,00 | 8,50 | | |
| Cocoamidopropyl Betaine | 4,00 | 1,60 | 3,00 | 3,00 |
| Ammonium Lauryl Sulfate | 4,00 | | | |
| Sodium Coco-Sulfate | | | 5,00 | 5,00 |
| Disodium Cocoamphodiacetate | | 1,00 | | |
| Cocamide MEA | 0,80 | 0,45 | | |
| PEG-7 Glyceryl Cocoate | 1,00 | 0,10 | | |
| PEG-40 Hydrogenated Castor Oil | | 0,30 | | |
| PEG-120 Methyl Glucose Dioleate | | 0,20 | | |
| Glycerin | | | 2,00 | 2,00 |
| Coco-Glucoside | | | 2,50 | 2,50 |
| Betaine | | | 1,00 | 1,00 |
| Caprylyl/Capryl Glucoside | | | 0,60 | 0,60 |
| Nicotinsäureamid | 3,00 | | | |
| Panthenol | 0,50 | | | |
| Polyglyceryl-3 Betainate Acetate | 0,30 | 0,20 | 0,50 | 0,50 |
| Propanediol Caprylate | 1,00 | | 0,50 | 0,50 |
| Carnithine | | 0,40 | | |
| Zinkoxid | | | | 1,00 |
| Natriumchlorid | 1,00 | 1,00 | 1,00 | 1,00 |
| Wasser, Konservierunq Parfümöle | ad 100 | ad 100 | ad 100 | ad 100 |

### Haarconditioner:

| | **9 [%]** | **10 [%]** | **11 [%]** | **12 [%]** | **13 [%]** | **14 [%]** |
|---|---|---|---|---|---|---|
| Polyglyceryl-3 betainate acetate* | 0,3 | 0,3 | 0,3 | 0,3 | 0,3 | |
| Steardimonium Hydroxypropyl Hydrolyzed Keratin | 0,2 | 0,2 | 0,2 | 0,2 | | |
| Hydrolyzed Keratin | 0,4 | 0,5 | 0,5 | 0,5 | | |
| Quaternium-87 | 0,75 | | | | 0,75 | |
| Glycoldistearat | 1,0 | | | | 1,0 | |
| Cetearylalkohol | 5,0 | | | | 5,0 | 4,0 |
| Guar Hydroxypropyltrimonium Chloride | 0,2 | | | | 0,4 | |
| Behenoyl-PG-Trimoniumchlorid | 1,5 | | | | 1,5 | |
| Distearoylethylhydroxyethylmoniummethosulfat | 0,3 | | | | 0,3 | |
| Behentrimoniumchlorid | 0,4 | | | | 0,4 | |
| Propandiol Caprylate | 1,0 | | | | 1,0 | |
| Glycerin | | 0,75 | 0,75 | 0,75 | | 4,0 |
| Distearoylethyl Dimonium Chloride | | | 0,8 | 0,8 | | 2,0 |
| Coco-Caprylate | | | 1,0 | | | 1,5 |
| Amodimethicone | | | | 1,0 | | 0,5 |
| Sorbitan Caprylate | | 1,0 | 1,0 | 1,0 | | 0,3 |
| Betaine | | 5,0 | 5,0 | 5,0 | | 0,25 |
| Arachidylalkohol | | 0,3 | 0,3 | 0,3 | | |
| Carnithin | | 0,4 | 0,4 | 0,4 | | 0,4 |
| Wasser, Konservierungsmittel, Parfüm, pH-Stellemittel | auf 100 | auf 100 | auf 100 | auf 100 | auf 100 | auf 100 |

| | | | | | | |
|---|---|---|---|---|---|---|
| * erhältlich bei Socri als Greenquat^{™} BT | | | | | | |

## Patentansprüche

1. Haarbehandlungsmittel enthaltend
a) mindestens ein Reaktionsprodukt, erhältlich durch Umsetzung von
i) Polyglycerin oder einem Polyglycerinester,
ii) Trimethylglycin und
iii) mindestens einer Säure, und
b) mindestens einen Aktivstoff.

2. Haarbehandlungsmittel nach Anspruch 1, wobei das Reaktionsprodukt a) erhältlich ist durch Reaktion von
i) Polyglycerine-3 und/oder Polyglyceryl-3 Oleate,
ii) Trimethylglycin und
iii) Essigsäure.

3. Haarbehandlungsmittel enthaltend
A) mindestens eine Verbindung der allgemeinen Formel (I)
R¹(OCH₂CH(OR²)CH₂)ₓ-O-R³ (I)
in der
- R¹, R², R³ unabhängig voneinander für -H, -C(O)-CH₂-N⁺(CH₃)₃ oder für einen geradkettigen oder verzweigten, gesättigten oder ungesättigten, -OH oder NH₂-Substituenten tragenden C₆-C₃₀-Acylrest stehen,
- x für eine Zahl von 1,1 bis 30 steht,
- wobei mindestens einer der Reste R¹, R², R³ für die Gruppierung -C(O)CH₂-N⁺(CH₃)₃ steht, und
- wobei Formel (I), eine der Anzahl der darin enthaltenen positiven Ladungen entsprechende Anzahl an physiologisch akzeptablen Anionen A⁻ enthält, und
B) mindestens einen Aktivstoff.

4. Haarbehandlungsmittel nach einem der Ansprüche 1 bis 3, enthaltend mindestens einen Betainester der allgemeinen Formel (la)
[R¹(OCH₂CH(OR²)CH₂)ₓ-O-C(O)-CH₂-N⁺(CH₃)₃] A⁻ (la)
in der
- R¹ für-H oder für Caprylat, Caprat, Laurat, Myristat, Palmitat, Stearat, Isostearat, Oleat, Ricinoleat, Arachidat, Behenat,
- R² für -H,
- x für eine Zahl von 1,1 bis 10, vorzugsweise von 1,1 bis 6, besonders bevorzugt von 2 bis 4 und insbesondere für 3 und
- A⁻ für ein physiologisch akzeptables organisches Anion wie Formiat, Acetat, Propionat, Benzoat, Citrat, Oxalat, Tartat, (Poly)acrylat, Fumarat, Maleat, Lactat, Itaconat, Glykolat, Gluconat, Malat, Mandelat, Tiglat, Succinat, Ascorbat steht.

5. Haarbehandlungsmittel nach einem der vorhergehenden Ansprüche, enthaltend als Komponente(n) a) oder A) eine unter der INCI-Bezeichnung Polyglyceryl-3 betainate acetate bekannte Verbindung.

6. Haarbehandlungsmittel nach einem der vorhergehenden Ansprüche, enthaltend die Komponente(n) a) oder A) in einer Menge von 0,01 bis 10,0 Gew.-% am Gesamtgewicht des Haarbehandlungsmittels.

7. Haarbehandlungsmittel nach einem der vorhergehenden Ansprüche, wobei der mindestens eine Aktivstoff ausgewählt ist aus der Gruppe bestehend aus einem Xanthin, einem Taurin, einem Vitamin, einem Antischuppenmittel, einem Echinacea-Pflanzenextrakt, einem Mikrobiom-Aktivmittel und einer Kombination davon.

8. Haarbehandlungsmittel nach einem der vorhergehenden Ansprüche, wobei Komponente(n) b) oder B) ausgewählt ist/sind aus der Gruppe bestehend aus Coffein, einem Echinacea-Extrakt, einem Taurin, einem anorganischen Zinksalz, Carnitin oder einer Kombination davon.

9. Haarbehandlungsmittel nach einem der vorhergehenden Ansprüche, enthaltend die Komponente(n) b) oder B) in einer Menge von 0,001 bis 5 Gew.-% am Gesamtgewicht des Haarbehandlungsmittels.

10. Haarbehandlungsmittel nach einem der vorhergehenden Ansprüche, das ein Haarshampoo ist und - bezogen auf das Gesamtgewicht des Haarshampoos - 1,0 bis 50,0 Gew.-% mindestens eines anionischen, nichtionischen, amphoteren und/oder zwitterionischen Tensids umfasst.

11. Haarbehandlungsmittel nach einem der vorhergehenden Ansprüche, das ein Haarkonditioniermittel ist und - bezogen auf das Gesamtgewicht des Haarbehandlungsmittels - 0,1 bis 15 Gew.-% mindestens eines kationischen Tensids umfasst.

12. Kosmetisches Verfahren zur Verbesserung der Haarpflege, insbesondere zur Verbesserung
- der antistatischen Eigenschaften,
- der Weichheit,
- der Kämmbarkeit und/oder Entwirrbarkeit von Haaren,
bei dem eine Zusammensetzung nach einem der Ansprüche 1 bis 11 auf die Haare aufgetragen wird.

13. Verwendung eines Haarbehandlungsmittels nach einem der Ansprüche 1 bis 11 zur Verbesserung der Haarpflege.
